(19) Europäisches Patentamt
European Patent Office
Office européen des brevets

(11) EP 1 357 118 A1

(12) EUROPEAN PATENT APPLICATION
published in accordance with Art. 158(3) EPC

(43) Date of publication:
29.10.2003 Bulletin 2003/44

(21) Application number: 01272547.9

(22) Date of filing: 28.12.2001

(51) Int Cl.7: C07D 311/16, C07C 45/45, C07C 47/575, C07C 43/23, A01N 43/16, A01N 35/04, A01N 37/02, A01N 43/30

(86) International application number:
PCT/JP01/11633

(87) International publication number:
WO 02/053552 (11.07.2002 Gazette 2002/28)

(84) Designated Contracting States:
AT BE CH CY DE DK ES FI FR GB GR IE IT LI LU MC NL PT SE TR
Designated Extension States:
AL LT LV MK RO SI

(30) Priority: 28.12.2000 JP 2000402725

(71) Applicant: Kureha Chemical Industry Co., Ltd.
Tokyo 103-8552 (JP)

(72) Inventors:
- NIIMURA, Koichi
Saitama-shi, Saitama 336-0017 (JP)
- KATOHNO, Masataka
Iwaki-shi, Fukushima 971-8185 (JP)
- SAGAWA, Kazuhiko
Iwaki-shi, Fukushima 974-8233 (JP)

(74) Representative: Minderop, Ralph H., Dr. rer. nat.
Cohausz & Florack,
Patentanwälte,
Kanzlerstrasse 8a
40472 Düsseldorf (DE)

# (54) PROCESS FOR PREPARATION OF ESCULETIN COMPOUNDS, ESCULETIN COMPOUNDS AND INTERMEDIATES THEREOF, AND USE OF BOTH

(57) An esculetin compound, an intermediate thereof, a process for manufacturing an esculetin compound, and an antifungal composition for agriculture and horticulture and an herbicide comprising an esculetin compound or an intermediate thereof are provided. The process for manufacturing an esculetin compound is a low-cost, high-yield, and industrially practicable process, and comprises the following step.

A process for manufacturing an esculetin compound of the formula (2):

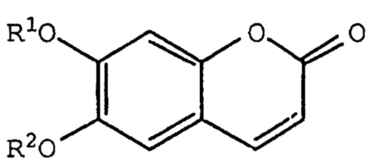

(2)

characterized by cyclizing a trihydroxybenzaldehyde compound of the formula (1):

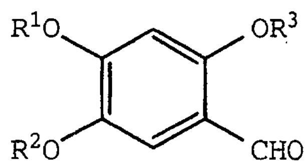

(1)

in an aprotic polar solvent in the presence of a weak base, with acetic anhydride or the like.

EP 1 357 118 A1

## Description

TECHNICAL FIELD

The present invention relates to an esculetin compound, an intermediate thereof, a process for manufacturing an esculetin compound, and an antifungal composition for agriculture and horticulture and an herbicide comprising an esculetin compound or an intermediate thereof, particularly a novel process for manufacturing an esculetin compound having an activity. Esculetin compounds are now important compounds in the pharmaceutical industry, and useful as a synthetic intermediate in the chemical industry.

BACKGROUND ART

Esculetin is a compound in which hydrogen atoms at 6- and 7- positions of coumarin are substituted with hydroxyl groups, i.e., 6,7-dihydroxycoumarin. Many methods for manufacturing coumarins are known, but there are few reports of a method of manufacturing esculetins having hydroxyl groups at 6- and 7- positions.

As a method for manufacturing esculetin per se, a method for preparation by hydrolyzing natural esculin (i. e., 6,7-dihydroxycoumarin-6-glucoside) by an acid [Merz, Arch. Pharm., vol. 270, 486(1932)], or a method by reacting 1,2,4-triacetoxybenzene with malic acid in sulfuric acid [Bull. Soc. Chim. France 512(1947), Heterocycle, vol. 41, 1979 (1995)] is known.

However, the method for hydrolyzing natural esculetin is limited in an amount supplied. Further, the method for synthesizing high-purity esculetin using 1,2,4-triacetoxybenzene and malic acid is industrially disadvantageous in view of a low yield (18%) and the use of 98% sulfuric acid.

As a method for manufacturing esculetin, a method for preparing esculetin by synthesizing esculetin having one or more substituents, and then by removing the substituents is known. In the method, 2-hydroxy-4,5-dimethoxy-benzaldehyde is reacted with malonic acid in pyridine in the presence of aniline to synthesize scoparone-3-carboxylic acid, the carboxyl group at 3-position is decarboxylated, and methyl ether is broken with hydriodic acid to synthesize esculetin [Phytochemistry, 27, 391(1988)]. As a method for synthesizing an esculetin derivative, a method for synthesizing scoparone by reacting an orthohydroxybenzaldehyde derivative with carbethoxymethylene triphenylphosphorane [Indian J. Chem., vol. 21B, 759(1982)], a method for synthesizing a coumarin derivative by reacting a salicylaldehyde derivative with carbethoxymethylene triphenylphosphorane [Chem. Pharm. Bull. vol. 40, 2614(1992)], a method for synthesizing a coumarin derivative by reacting a salicylaldehyde derivative with acetic anhydride and sodium acetate [Chem. Pharm. Bull. vol. 40, 2614(1992), Chem. Ber. vol. 32, 287(1899)], a method for synthesizing scoparone (6,7-dimethoxycoumarin) by reacting 2-hydroxy-4,5-dimethoxy-benzaldehyde with acetic anhydride and potassium acetate in the presence of hydroquinone [Japanese Unexamined Patent Publication (Kokai) No. 48-22466], or the like is known. The above methods are a method for synthesizing an esculetin derivative, but not a method for directly synthesizing esculetin per se. Therefore, a step of removing a protecting group or a substituent is necessary, and thus the yield becomes low.

In the above method for synthesizing 6-isopropoxy-7-methoxycoumarin by reacting a salicylaldehyde derivative with acetic anhydride and sodium acetate, the above reference discloses that the yield is 45.7%, which is not enough. Further, the yield of the above method for synthesizing scoparone by reacting 2-hydroxy-4,5-dimethoxy-benzaldehyde with acetic anhydride and potassium acetate in the presence of hydroquinone is 41%, which is not enough.

The present inventors have conducted intensive studies of a method for manufacturing an esculetin compound which can satisfy industrial requirements and, as a result, found that an esculetin compound can be obtained with a high yield, by cyclocondensing a specific benzaldehyde compound in an aprotic polar solvent (such as N,N-dimethylformamide or the like) in the presence of a weak base (such as sodium acetate or the like), with acetic anhydride or the like. Further, the present inventors found that novel compounds are included in starting materials, intermediate products, and products used in the above novel manufacturing method found by the present inventors. Furthermore, the present inventors found that compounds having an antifungal activity or an herbicidal activity are included in the intermediate products.

The present invention is based on the above findings.

DISCLOSURE OF INVENTION

Accordingly, the present invention relates to a process for manufacturing an esculetin compound of the general formula (2):

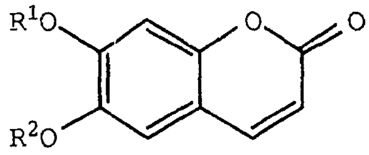

(2)

wherein $R^1$ and $R^2$ are, independently, a hydrogen atom, an alkyl group having 1 to 5 carbon atoms, an optionally substituted benzyl group, a formyl group, or an optionally substituted alkyl-carbonyl group in which the alkyl moiety has 1 to 4 carbon atoms, or $R^1$ and $R^2$ together form a $-CH_2-$ group or a $-C(CH_3)_2-$ group; characterized by cyclocondensing a trihydroxybenzaldehyde compound of the general formula (1):

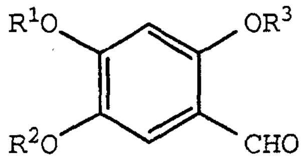

(1)

wherein $R^1$ and $R^2$ have the same meanings as above; and $R^3$ is a hydrogen atom, a formyl group, or an optionally substituted alkyl-carbonyl group in which the alkyl moiety has 1 to 4 carbon atoms; in an aprotic polar solvent in the presence of a weak base, with acetic anhydride or a compound which forms acetic anhydride in the reaction system.

The present invention relates to a process for manufacturing a trihydroxybenzaldehyde compound of the general formula (1), characterized by performing a formylation of a trihydroxybenzene compound of the general formula (3):

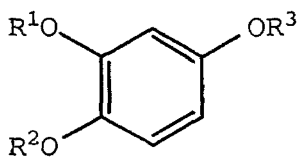

(3)

wherein $R^1$ and $R^2$ are, independently, a hydrogen atom, an alkyl group having 1 to 5 carbon atoms, an optionally substituted benzyl group, a formyl group, or an optionally substituted alkyl-carbonyl group in which the alkyl moiety has 1 to 4 carbon atoms, or $R^1$ and $R^2$ together form a $-CH_2-$ group or a $-C(CH_3)_2-$ group; and $R^3$ is a hydrogen atom, a formyl group, or an optionally substituted alkyl-carbonyl group, in which the alkyl moiety has 1 to 4 carbon atoms.

As the trihydroxybenzaldehyde compound of the general formula (1), a trihydroxybenzaldehyde compound of the general formula (1) wherein $R^1$ and $R^2$ are, independently, a hydrogen atom, an alkyl group having 1 to 5 carbon atoms, a benzyl group, or an acetyl group, or $R^1$ and $R^2$ together form a $-CH_2-$ group or a $-C(CH_3)_2-$ group, and $R^3$ is a hydrogen atom or an acetyl group is preferable.

As the esculetin compound of the general formula (2), an esculetin compound of the general formula (2) wherein $R^1$ and $R^2$ are, independently, a hydrogen atom, an alkyl group having 1 to 5 carbon atoms, a benzyl group, or an acetyl group, or $R^1$ and $R^2$ together form a $-CH_2-$ group or a $-C(CH_3)_2-$ group, and $R^3$ is a hydrogen atom or an acetyl group is preferable.

As the trihydroxybenzene compound of the general formula (3), a trihydroxybenzene compound of the general formula (3) wherein $R^1$ and $R^2$ are, independently, a hydrogen atom, an alkyl group having 1 to 5 carbon atoms, a benzyl group, or an acetyl group, or $R^1$ and $R^2$ together form a $-CH_2-$ group or a $-C(CH_3)_2-$ group, and $R^3$ is a hydrogen atom or an acetyl group is preferable.

The present invention relates to a trihydroxybenzaldehyde compound of the general formula (10):

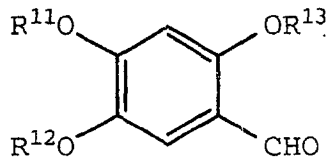

(10)

wherein $R^{11}$ and $R^{12}$ are, independently, a hydrogen atom or an alkyl group having 2 to 5 carbon atoms, or $R^{11}$ and $R^{12}$ together form a $-C(CH_3)_2-$ group; and $R^{13}$ is a hydrogen atom, a formyl group, or an optionally substituted alkyl-carbonyl group in which the alkyl moiety has 1 to 4 carbon atoms; with the proviso that $R^{11}$ and $R^{12}$ are not a hydrogen atom at the same time.

As the trihydroxybenzaldehyde compound of the general formula (10), a trihydroxybenzaldehyde compound of the general formula (10) wherein $R^{11}$ and $R^{12}$ are, independently, a hydrogen atom or an alkyl group having 2 to 5 carbon atoms, or $R^{11}$ and $R^{12}$ together form a $-C(CH_3)_2-$ group, and $R^{13}$ is a hydrogen atom or an acetyl group, with the proviso that $R^{11}$ and $R^{12}$ are not a hydrogen atom at the same time, is preferable.

The present invention relates to an esculetin compound of the general formula (20):

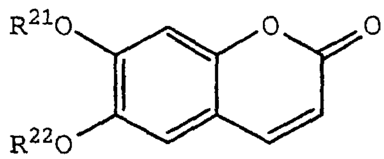

(20)

wherein $R^{21}$ and $R^{22}$ are, independently, a hydrogen atom, an alkyl group having 2 to 5 carbon atoms, an optionally substituted benzyl group, a formyl group, or an optionally substituted alkyl-carbonyl group in which the alkyl moiety has 1 to 4 carbon atoms, or $R^{21}$ and $R^{22}$ together form a $-C(CH_3)_2-$ group, with the proviso that $R^{21}$ and $R^{22}$ are not a hydrogen atom at the same time, and that $R^{21}$ and $R^{22}$ are not a formyl group, or an optionally substituted alkyl-carbonyl group in which the alkyl moiety has 1 to 4 carbon atoms at the same time.

As the esculetin compound of the general formula (20), an esculetin compound of the general formula (20) wherein $R^{21}$ and $R^{22}$ are, independently, a hydrogen atom, an alkyl group having 2 to 5 carbon atoms, a benzyl group, or an acetyl group, or $R^{21}$ and $R^{22}$ together form a $-C(CH_3)_2-$ group, with the proviso that $R^{21}$ and $R^{22}$ are not a hydrogen atom at the same time, and that $R^{21}$ and $R^{22}$ are not an acetyl group at the same time is preferable.

The present invention relates to a trihydroxybenzene compound of the general formula (30):

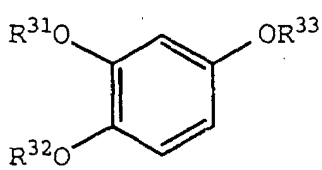

(30)

wherein $R^{31}$ is an optionally substituted benzyl group, $R^{32}$ is a hydrogen atom, a formyl group, or an optionally substituted alkyl-carbonyl group in which the alkyl moiety has 1 to 4 carbon atoms, or $R^{31}$ and $R^{32}$ together form a $-C(CH_3)_2-$ group; and $R^{33}$ is a hydrogen atom, a formyl group, or an optionally substituted alkyl-carbonyl group, in which the alkyl moiety has 1 to 4 carbon atoms.

As the trihydroxybenzene compound of the general formula (30), a trihydroxybenzene compound of the general formula (30) wherein $R^{31}$ is a benzyl group, $R^{32}$ is a hydrogen atom or an acetyl group, or $R^{31}$ and $R^{32}$ together form a $-C(CH_3)_2-$ group, and $R^{33}$ is a hydrogen atom or an acetyl group is preferable.

The present invention relates to an antifungal composition for agriculture and horticulture, characterized by comprising, as an active ingredient, the trihydroxybenzaldehyde compound of the general formula (10), the esculetin compound of the general formula (20), or the trihydroxybenzene compound of the general formula (30).

The present invention relates to an herbicide, characterized by comprising, as an active ingredient, the trihydroxybenzaldehyde compound of the general formula (10), the esculetin compound of the general formula (20), or the trihydroxybenzene compound of the general formula (30)

BEST MODE FOR CARRYING OUT THE INVENTION

In the process of the present invention for manufacturing an esculetin compound, the trihydroxybenzaldehyde compound of the general formula (1) is used as a starting material.

In the general formula (1), $R^1$ and $R^2$ may be the same or different, and $R^1$ and $R^2$ are, independently, a hydrogen atom, an alkyl group having 1 to 5 carbon atoms, an optionally substituted benzyl group, a formyl group, or an optionally substituted alkyl-carbonyl group in which the alkyl moiety has 1 to 4 carbon atoms, or $R^1$ and $R^2$ together form a $-CH_2-$ group or a $-C(CH_3)_2-$ group, and $R^3$ is a hydrogen atom, a formyl group, or an optionally substituted alkyl-carbonyl group in which the alkyl moiety has 1 to 4 carbon atoms.

The term "alkyl group" as used herein means a straight-chain alkyl group, a branched alkyl group, or a cyclic alkyl group (a cycloalkyl group), or alkyl group comprising a cyclic moiety (a cycloalkyl-alkyl group or an alkyl-cycloalkyl group). As the alkyl group having 1 to 5 carbon atoms, there may be mentioned, for example, methyl, ethyl, n-propyl, isopropyl, n-butyl, isobutyl, sec-butyl, tert-butyl, n-pentyl, isopentyl, 1-methyl-butyl, 2-methyl-butyl, cyclopropyl, cyclobutyl, cyclopentyl, 2-cyclopropyl-ethyl, or 1-cyclobutyl-methyl.

The benzyl group as used herein may be unsubstituted or have one or more substituents. When the benzene group has one or more substituents, the benzene group may be substituted with 1 to 5 substituents (preferably 1 to 2 substituents) of an alkyl group having 1 to 4 carbon atoms, an alkoxy group having 1 to 4 carbon atoms, and/or a halogen atom (such as fluorine, chlorine, bromine, or iodine). These substituents preferably exist on the phenyl group.

The alkyl-carbonyl group in which the alkyl moiety has 1 to 4 carbon atoms (hereinafter referred to as $C_{1-4}$ alkyl-carbonyl group) as used herein may be unsubstituted or have one or more substituents. The alkyl moiety having 1 to 4 carbon atoms in a substituted $C_{1-4}$ alkyl-carbonyl group may be substituted with one or more alkoxy groups having 1 to 4 carbon atoms and/or halogen atoms (fluorine, chlorine, bromine, or iodine). When the alkyl moiety of the $C_{1-4}$ alkyl-carbonyl group is substituted with one or more alkoxy groups having 1 to 4 carbon atoms, the number of the substituents is preferably 1 to 2.

When the alkyl moiety of the $C_{1-4}$ alkyl-carbonyl group is substituted with one or more halogen atoms, it may be substituted with one to a certain number (i.e., a number of substitutable hydrogen atoms) of fluorine, chlorine, bromine, and/or iodine groups. When it is substituted with a kind of halogen atom, the number of substituted fluorine atoms is preferably 1 to a number of substitutable hydrogen atoms, the number of substituted chlorine atoms is preferably 1 to 3, and the number of substituted bromine or iodine atoms is preferably 1 to 2. When it is substituted with plural kinds of halogen atoms, it is preferable that each of the above preferable number in the case of substitution with a kind of halogen atom is combined, within a number of substitutable hydrogen atoms.

When it is substituted with plural kinds of substituents, it is preferable that each of a preferable number of substitutions is combined.

In the general formula (1), it is preferable that $R^1$ is a methyl group, a benzyl group, or an acetyl group, $R^2$ is a hydrogen atom or an acetyl group, and $R^3$ is a hydrogen atom or an acetyl group. Further, it is more preferable that $R^1$ is a benzyl group, $R^2$ is a hydrogen atom or an acetyl group, and $R^3$ is a hydrogen atom or an acetyl group.

As concrete examples of the trihydroxybenzaldehyde compound of the general formula (1), there may be mentioned, for example,
2,4,5-trihydroxybenzaldehyde,
4-methyloxy-2,5-dihydroxybenzaldehyde,
4-ethyloxy-2,5-dihydroxybenzaldehyde,
4-propyloxy-2,5-dihydroxybenzaldehyde,
4-butyloxy-2,5-dihydroxybenzaldehyde,
4-benzyloxy-2,5-dihydroxybenzaldehyde,
4,5-dimethyloxy-2-hydroxybenzaldehyde,
4,5-methylenedioxy-2-hydroxybenzaldehyde, or
5-hydroxy-6-formyl-2,2-dimethyl-1,3-benzodioxole, or acetylated compounds of the above compounds, such as
2,4,5-triacetoxybenzaldehyde,
2-acetoxy-4,5-hydroxybenzaldehyde,
2,5-diacetoxy-4-benzyloxybenzaldehyde, or the like.

The process of the present invention for manufacturing an esculetin compound is carried out in an aprotic

polar solvent. The aprotic polar solvent is not particularly limited, so long as it is inert in the reaction system of the present invention. As the aprotic polar solvent, there may be mentioned, for example, N,N-dimethylformamide, N,N-diethylformamide, N,N-dimethylacetamide, N,N-diethylacetamide, 1-methyl-2-pyrrolidinone, 1,1,3,3,-tetramethylurea, or 1,3-dimethyl-2-imidazolidinone. N,N-dimethylformamide, N,N-diethylformamide, 1,1,3,3,-tetramethylurea, or 1,3-dimethyl-2-imidazolidinone may be preferably used.

The process of the present invention for manufacturing an esculetin compound is carried out in the presence of a weak base. The weak base is not particularly limited, but there may be mentioned, for example, alkali metal salts (such as sodium acetate or potassium acetate) of organic acids (such as carboxylic acid or oxalic acid), alkali metal salts (such as potassium carbonate, sodium hydrogencarbonate, or lithium carbonate) of inorganic acids, or N-containing aromatic heterocyclic compound (such as pyridine, quinoline, or dimethylaminopyridine.

In the process of the present invention for manufacturing an esculetin compound, the trihydroxybenzaldehyde compound of the general formula (1) is cyclocondensed by acetic anhydride or a compound which forms acetic anhydride in the reaction system.

The term "compound which forms acetic anhydride in the reaction system" as used herein is a compound which reacts with the weak base to form acetic anhydride in a step of the process of the present invention for manufacturing an esculetin compound. As the compound, there may be
mentioned, for example, acetyl halide (such as acetyl chloride or acetyl bromide).

When acetic anhydride is used in the process of the present invention for manufacturing an esculetin compound, with respect to the trihydroxybenzaldehyde compound of the general formula (1), 0.5 to 8 equivalents (preferably 3 to 4 equivalents) of acetic anhydride may be used in the presence of 1 to 10 equivalents (preferably 3 to 4 equivalents) of the weak base (such as sodium acetate).

When the compound which forms acetic anhydride in the reaction system (such as acetyl halide) is used, with respect to the trihydroxybenzaldehyde compound of the general formula (1), 1 to 10 equivalents (preferably 3 to 4 equivalents) of the compound which forms acetic anhydride (such as acetyl halide) may be used in the presence of 2 to 10 equivalents (preferably 3 to 4 equivalents) of the weak base (such as sodium acetate).

A feature of the process of the present invention is that, even if a trihydroxybenzaldehyde compound in which one or more acetoxy groups bind to 3- and/or 4-positions is used as a starting material, an esculetin compound in which one or more acetoxy groups at 6- and/or 7-positions of coumarin are converted to hydroxyl groups can be obtained, as a product, by using preferable reaction conditions (or more preferable reaction conditions) described in the present specification.

The process of the present invention for manufacturing an esculetin compound may be carried out under atmospheric conditions, but preferably in an inert gas (such as a nitrogen gas or an argon gas) atmosphere. The reaction temperature and the reaction time may be changed in accordance with a starting material used, but the reaction is carried out while heating at preferably 100 to 200°C, more preferably 150 to 180°C for generally 1 to 24 hours, preferably 2 to 5 hours.

The trihydroxybenzaldehyde compound of the general formula (1), which is used as a starting material in the process of the present invention for manufacturing an esculetin compound, can be manufactured by performing a formylation of the trihydroxybenzene compound of the general formula (3).

The formylation can be carried out by a known method per se, for example, by treating it with an agent for formylation, if necessary in the presence of an appropriate catalyst. As the agent for formylation, for example, trialkyl orthoformate may be used. As the catalyst, for example, aluminum halide may be used.

The formylation is preferably carried out in an ethereal or aromatic organic solvent. More particularly, ether, dimethoxyethane, dioxane, tetrahydrofuran, toluene, ethylbenzene, xylene, or chlorobenzene may be preferably used. The formylation may be carried out under atmospheric conditions, but preferably in an inert gas (such as a nitrogen gas or an argon gas) atmosphere. The reaction temperature and the reaction time may be changed in accordance with a starting material used, but the reaction is carried out at preferably 0 to 40°C, more preferably 0 to 10°C for generally 0.1 to 10 hours, preferably 0.1 to 1 hour.

Among the trihydroxybenzene compound of the general formula (3) as one of intermediates of esculetin compounds of the present invention used in the manufacture of the trihydroxybenzaldehyde compound of the general formula (1), the trihydroxybenzene compound of the general formula (30) is a novel compound. The trihydroxybenzene compound of the general formula (30) can be prepared by a known method per se.

For example, the compound of the general formula (30) wherein $R^{31}$ is a benzyl group, $R^{32}$ is an acetyl group, and $R^{33}$ is a hydrogen atom or an acetyl group can be obtained, for example, by oxidizing known 2-benzyloxyphenol, using a Fremy salt (i.e., potassium nitrososulfonate) or by bubbling oxygen in the presence of a cobalt complex [Tetrahedron Letters, vol. 30, 5929(1989)], to form 2-benzyloxy-1,4-benzoquinone, and then by performing reductive acetoxylation of 2-benzyloxy-1,4-benzoquinone with zinc powder or the like in the presence of acetic anhydride and pyridine. In this connection, the acetyl group can be easily hydrolyzed with hydrochloric acid or the like.

Further, the compound of the general formula (30) wherein $R^{31}$ and $R^{32}$ together form a $-CH_2-$ group or a -C

$(CH_3)_2$- group, and $R^{33}$ is a hydrogen atom or an acetyl group can be obtained, for example, by reacting 1,2,4-trihydroxybenzene with dichloromethane and cesium fluoride in dimethylformamide, or by reacting 1,2,4-trihydroxybenzene with 2,2-dimethoxypropane in the presence of an acid catalyst.

Among the trihydroxybenzaldehyde compound of the general formula (1) as one of intermediates of esculetin compounds of the present invention used as a starting material in the process of the present invention for manufacturing an esculetin compound, the trihydroxybenzaldehyde compound of the general formula (10) is a novel compound. The trihydroxybenzaldehyde compound of the general formula (10) can be prepared by a known method per se.

For example, the trihydroxybenzaldehyde compound of the general formula (10) may be prepared by performing a formylation of a corresponding trihydroxybenzene compound. More particularly, for example, the compound of the general formula (10) wherein $R^{11}$ is an alkyl group having 2 to 5 carbon atoms, $R^{12}$ is a hydrogen atom, and $R^{13}$ is a hydrogen atom can be easily obtained by performing a formylation of known 2-alkyloxyhydroquinone with triethyl orthoformate ester or the like in the presence of aluminum chloride. Further, the compound of the general formula (10) wherein $R^{11}$ and $R^{12}$ together form a $-C(CH_3)_2$- group, and $R^{13}$ is a hydrogen atom may be easily by performing a formylation of 5-hydroxy(or acetyloxy)-2,2-dimethyl-1,3-benzodioxol in a similar fashion.

In this connection, the compound in which $R^{13}$ is an acetyl group can be obtained by acetylating the resulting product in the accordance with a conventional method.

Among the trihydroxybenzaldehyde compounds of the general formula (1), methods for manufacturing typical known compounds will be explained. For example, 2,4,5-trihydroxybenzaldehyde, which corresponds to the compound of the general formula (1) wherein $R^1$ is a hydrogen atom, is commercially available (TOKYO KASEI KOGYO CO., LTD. or the like), or may be easily obtained by performing a formylation of commercially available 1,2,4-trihydroxybenzene with triethyl orthoformate ester or the like in the presence of aluminum chloride. Further, 4-methyloxy-2,5-dihydroxybenzaldehyde, which corresponds to the compound of the general formula (1) wherein $R^1$ is a methyl group, may be easily obtained by performing a formylation of commercially available 2-methyloxy-hydroquinone in a similar fashion. Furthermore, 4-benzyloxy-2,5-dihydroxybenzaldehyde, which corresponds to the compound of the general formula (1) wherein $R^1$ is a benzyl group, may be easily obtained by performing benzylation of 2,4,5-hydroxybenzaldehyde, or by performing a formylation of 2-benzyloxyhydroquinone.

Further, 4,5-dimethyloxy-2-hydroxybenzaldehyde, which corresponds to the compound of the general formula (1) wherein $R^1$ and $R^2$ are methyl groups, may be easily obtained by performing a formylation of 3,4-dimethoxyphenol in a similar fashion. Furthermore, 4,5-methylenedioxy-2-hydroxybenzaldehyde, which corresponds to the compound of the general formula (1) wherein $R^1$ and $R^2$ together form a $-CH_2$- group, may be easily obtained by performing a formylation of commercially available sesamol in a similar fashion.

Further, 2,4,5-triacetoxybenzaldehyde, 2-acetoxy-4,5-hydroxybenzaldehyde, or 2,5-diacetoxy-4-benzyloxybenzaldehyde, which corresponds to the compound of the general formula (1) wherein $R^1$, $R^2$, and/or $R^3$ are acetyl groups, may be obtained by acetylating 2,4,5-trihydroxybenzaldehyde or 4-benzyloxy-2,5-dihydroxybenzaldehyde with acetic anhydride and sodium acetate or with acetyl chloride and sodium acetate.

Among the esculetin compound of the general formula (2) obtained in the process of the present invention for manufacturing an esculetin compound, the esculetin compound of the general formula (20) is a novel compound. The novel esculetin compound of the general formula (20) can be prepared by the process of the present invention for manufacturing an esculetin compound.

The trihydroxybenzaldehyde compound of the general formula (10), the esculetin compound of the general formula (20), and the trihydroxybenzene compound of the general formula (30) exhibit an antifungal activity and an herbicidal activity, and thus can be used as an active ingredient of an antifungal agent for agriculture and horticulture or an herbicide.

When the above compound is used as an antifungal agent for agriculture and horticulture or an antifungal composition for agriculture and horticulture, or an herbicide or an herbicide composition, the compound may be used alone, but is generally formulated and used with one or more adjuvants in various forms, such as dust, water dispesible powder, granules, emulsifiable concentrate, or the like. The formulation is prepared so that it contains preferably 0.1 to 95 weight%, more preferably 0.5 to 90 weight%, most preferably 2 to 70 weight% of one or more kinds of the trihydroxybenzaldehyde compound of the general formula (10), the esculetin compound of the general formula (20), and/or the trihydroxybenzene compound of the general formula (30).

A carrier, a diluent, or a surfactant which can be used as the adjuvant is as follows. As a solid carrier, there may be mentioned, for example, talc, kaolin, bentonite, diatomaceous earth, white carbon, clay, or the like. As a liquid diluent, there may be mentioned, for example, water, toluene, xylene, cyclohexane, chlorobenzene, lower alcohols, cyclohexanone, N,N-dimethylformamide, N-methylpyrrolidone, dimethylsulfoxide, or the like. As the surfactant, it is preferable to select an appropriate surfactant in accordance with its effect. For example, as an emulsifying agent, polyoxyethylene alkyl aryl ether, polyoxyethylene sorbitan monolaurate, or the like is preferable. As a dispersing agent, lignin sulfonate, dibutylnaphthalene sulfonate, or the like is preferable. As a wetting agent, there may be mentioned, for example, alkyl sulfonate, alkylphenyl sulfonate, or the like.

The antifungal agent for agriculture and horticulture or the antifungal composition for agriculture and horticulture, or the herbicide or the herbicide composition can be used directly, or after diluting it with a diluent such as water or the like to a predetermined concentration. When it is diluted and used, the concentration of the trihydroxybenzaldehyde compound of the general formula (10), the esculetin compound of the general formula (20), and the trihydroxybenzene compound of the general formula (30) is preferably 0.001 to 1.0%. The amount to be used of the trihydroxybenzaldehyde compound of the general formula (1.0), the esculetin compound of the general formula (20), and the trihydroxybenzene compound of the general formula (30) may be appropriately selected in accordance with conditions of cultivation. It is preferably 20 to 5000 g, more preferably 50 to 1000 g per 1 ha of a land for agriculture and horticulture, such as a field, a paddy field, a fruit farm, a greenhouse, or the like. The concentration and the amount to be used will be changed in accordance with a form, a season, a method, a place, or a plant, and thus it can be increased or decreased beyond the above range. The trihydroxybenzaldehyde compound of the general formula (10), the esculetin compound of the general formula (20), and the trihydroxybenzene compound of the general formula (30) may be used in combination with other active ingredients, such as a bactericide, an insecticide, a tickicide, an herbicide, a fertilizer, or an ameliorant.

A method and a period for application are not particularly limited. For example, it can be sprayed on the soil before or after seeding of the plant, or after germination or growth of the plant.

EXAMPLES

The present invention now will be further illustrated by, but is by no means limited to, the following Examples.

Example 1: Synthesis of esculetin

The reaction procedure of the present example is shown as follows. In the following reaction procedures, DMF is dimethylformamide, and $Ac_2O$ is acetic anhydride.

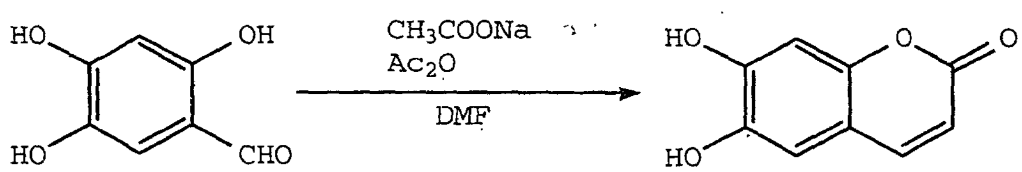


The actual procedures are as follows.

To an eggplant type flask (50 mL), 2,4,5-trihydroxybenzaldehyde (2.6 g, 16.9 mmol, 1.0 equivalent), sodium acetate (4.15 g, 50.7 mmol, 3.0 equivalents), acetic anhydride (6.4 mL, 67.6 mmol, 4.0 equivalents), and dried DMF (26 mL) were added. The mixture was stirred in a nitrogen atmosphere at 180°C for 5 hours, whereupon the starting materials were completely dissolved. The heating was stopped and the mixture was cooled to room temperature.

The reaction liquid was poured into a 10% hydrochloric acid aqueous solution (100 mL), and extracted with ethyl acetate (60 mL×10). The resulting organic layer was washed with distilled water (150 mL×1) and saturated brine (150 mL×1), and dried over sodium sulfate.

The solvent was evaporated under a reduced pressure, and ethyl acetate (10 mL) was added. The precipitated crystals were collected to obtain esculetin (2.16 g, yield = 80%) as pale brown crystals.
TLC: Rf = 0.57 ($CHCl_3$:MeOH:$CH_3COOH$:$H_2O$ = 16:8:1:2)
$^1$H-NMR ($d_6$-DMSO, $\delta$ ppm): 6.15 (d, 1H, J=9.3Hz, H-3), 6.72 (s, 1H, H-5), 6.96 (s, 1H, H-8), 7.85 (d, 1H, J=9.3Hz, H-4)
IR (KBr $cm^{-1}$): 3232, 2364, 1672, 1626, 1564, 1524, 1460, 1404, 1282, 1200, 1148, 946, 878, 848, 820, 748, 670, 636, 576, 508, 454, 418

Example 2: Synthesis of 6-hydroxy-7-methyloxycoumarin (isoscopoletin)

The reaction procedure of the present example is shown as follows. In the following reaction procedures, Me is a methyl group, and AcCl is acetyl chloride.

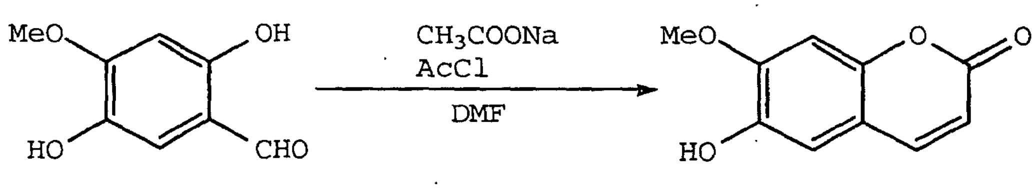


The actual procedures are as follows.

To an eggplant type flask, a mixture of sodium acetate (443 mg, 5.4 mmol, 5.0 equivalents) and dried DMF (26 mL) was added. Acetyl chloride (0.23 mL, 3.24 mmol, 3.0 equivalents) was added dropwise in a nitrogen gas stream under stirring while cooling on ice. After stirring at room temperature for 10 minutes, a solution of 2,5-dihydroxy-4-methoxybenzaldehyde (167 mg, 1.08 mmol, 1.0 equivalent) in dimethylformamide (DMF) (0.66 mL×3) was added dropwise to the reaction system. The mixture was stirred in a nitrogen atmosphere at 180°C for 2 hours, whereupon the starting materials were completely dissolved. The heating was stopped and the mixture was cooled to room temperature.

The reaction liquid was poured into a 10% hydrochloric acid aqueous solution (30 mL) while cooling on ice, and extracted with ethyl acetate (20 mL×6). The resulting organic layer was washed with distilled water (50 mL×3) and saturated brine (50 mL×3), and dried over sodium sulfate.

The solvent was evaporated under a reduced pressure, and the precipitated crystals were washed with toluene, and then collected to obtain isoscopoletin (154 mg, yield = 79%) as pale brown crystals.
TLC: Rf = 0.29 (n-hexane/ethyl acetate = 1:1)
$^1$H-NMR ($CDCl_3$, δ ppm): 3.90 (s, 3H, $CH_3O$), 6.18 (d, 1H, J=9.3Hz, H-3), 6.90 (s, 1H, H-5), 6.91 (s, 1H, H-8), 7.75 (d, 1H, J=9.3Hz, H-4)

Example 3: Synthesis of 7-benzyloxy-6-hydroxycoumarin (7-benzylesculetin)

The reaction procedure of the present example is shown as follows. In the following reaction procedures, Bn is a benzyl group.

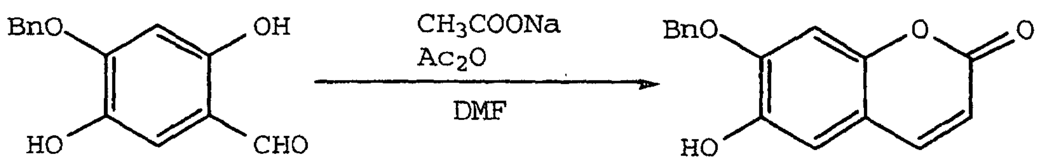


The actual procedures are as follows.

To an eggplant type flask (50 mL), 4-benzyloxy-2,5-dihydroxybenzaldehyde (488.48 mg, 2.0 mmol, 1.0 equivalent), sodium acetate (492.2 mg, 6.0 mmol, 3.0 equivalents), acetic anhydride (816.8 mg, 8.0 mmol, 4.0 equivalents), and dried DMF (10 mL) were added. The mixture was stirred in a nitrogen atmosphere at 180°C for 5 hours, whereupon the starting materials were completely dissolved. The heating was stopped and the mixture was cooled to room temperature.

The reaction liquid was poured into a 1% hydrochloric acid aqueous solution (20 mL), and extracted with ethyl acetate (100 mL×1). The resulting organic layer was washed with distilled water (20 mL×3) and saturated brine (20 mL×3), and dried over sodium sulfate (10 g) to obtain a crude product (652.2 mg).

The solvent was evaporated under a reduced pressure, and the resulting residue was purified by silica gel chromatography [3.0(diameter)×7 cm, 20 g; n-hexane:ethyl acetate (5:1-2:1); 300 mL + 120 mL] to obtain 7-benzylesculetin (390 mg, 72.7%) as pale brown crystals (yield: 72.7%).
TLC: Rf = 0.15 (n-hexane/ethyl acetate = 2:1)
$^1$H-NMR ($CDCl_3$, δ ppm): 5.19 (s, 2H, $CH_2Ph$), 5.66 (s, 1H, OH), 6.29 (d, J=9.77Hz, 1H, H-3), 6.90 (s, 1H, Ar-H), 6.99 (s, 1H, Ar-H), 7.3-7.4 (br, 1H, Ph), 7.60 (d, J=9.77Hz, 1H, H-4)
IR (KBr $cm^{-1}$): 3232, 2304, 1694, 1624, 1566, 1512, 1472, 1456, 1402, 1386, 1360, 1320, 1292, 1210, 1150, 1106, 986, 942, 888, 828, 744, 700, 630, 594, 572, 478

Esculetin can be quantitatively obtained by hydrogenolyzing the resulting 7-benzylesculetin with hydrogen in the presence of 10% Pd/C in methanol.

Example 4: Synthesis of esculetin

The reaction procedure of the present example is shown as follows.

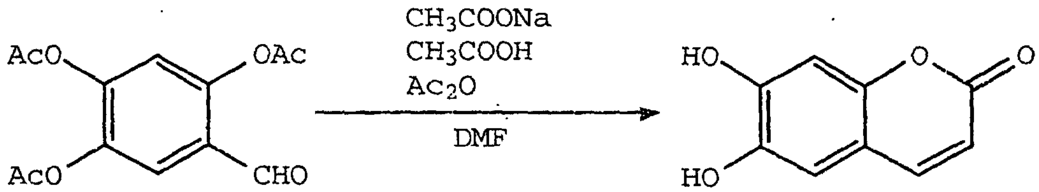


The actual procedures are as follows.

To a solution of 2,4,5-triacetoxybenzaldehyde (455 mg, 1.62 mmol) in DMF (9.0 mL, 0.18 mol/l), acetic acid (0.19 mL, 2.0 equivalents), sodium acetate (267 mg, 2.0 equivalents), and acetic anhydride (0.18 mL, 1.2 equivalents) were added, and the mixture was reacted at 180°C for 5 hours. After dissolution of the starting materials was confirmed by thin layer chromatography (TLC), a 10% hydrochloric acid aqueous solution (100 mL) was added, and then the reaction mixture was extracted with ethyl acetate (80 mL×8). The combined organic layer was washed with distilled water (100 mL) and saturated brine (100 mL), dried over sodium sulfate, and then concentrated under a reduced pressure (azeotropy with toluene) to obtain brown crystals (301 mg). The content of esculetin was found to be 74%, by determining the crystals by HPLC. Therefore, the actual yield of esculetin was 75% by calculation.

Example 5: Synthesis of 7-benzyloxy-6-hydroxycoumarin

The reaction procedure of the present example is shown as follows. In the following reaction procedures, Bn is a benzyl group, and AcCl is acetyl chloride.

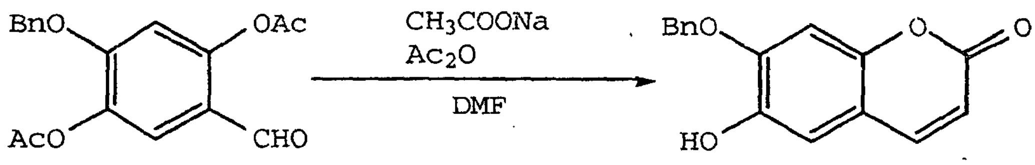


The actual procedures are as follows.

To an eggplant type flask (25 mL), 4-benzyloxy-2,5-dihydroxybenzaldehyde (164 mg, 0.5 mmol, 1.0 equivalent), sodium acetate (82 mg, 1 mmol, 2.0 equivalents), and dried DMF (4 mL) were added. To the mixture, a solution of acetic anhydride (204 mg, 2 mmol, 4.0 equivalents) in DMF (1.5 mL) was slowly added dropwise at room temperature. The mixture was stirred in a nitrogen atmosphere at 180°C for 4 hours. After the reaction, the reaction liquid was cooled to room temperature.

To the reaction liquid; ethyl acetate (60 mL) and water (30 mL) were added and widely distributed. After separating an aqueous layer, the resulting organic layer was washed with saturated brine (10 mL×3), and dried over sodium sulfate (5 g).

The solvent was evaporated under a reduced pressure, and the resulting residue (191.8 mg) was purified by silica gel chromatography [2.5 (diameter) ×2 cm, 5 g; n-hexane:ethyl acetate (1:2)] to obtain 7-benzylesculetin (119.4 mg).

Brown and powdery crystals (100.3 mg ,yield = 74.7%) were obtained by washing with n-hexane.

TLC: Rf = 0.18 (n-hexane/ethyl acetate = 2:1)

Esculetin can be quantitatively obtained by hydrogenolyzing the resulting 7-benzylesculetin with hydrogen in the presence of 10% Pd/C in methanol.

Example 6

(1) Synthesis of methylenedioxycoumarin

The reaction procedure of synthesis of methylenedioxycoumarin is shown as follows.

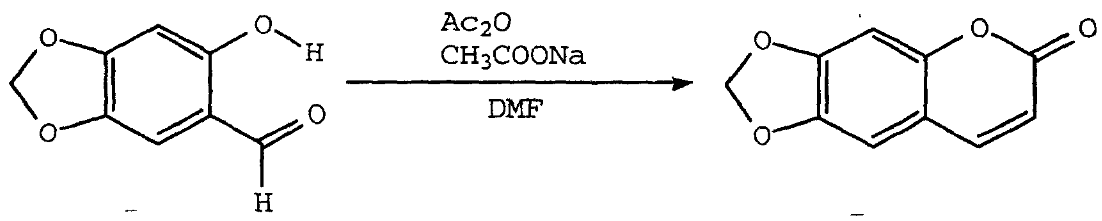


The actual procedures are as follows.

To an eggplant type flask (25 mL), 4,5-methylenedioxysalicylaldehyde (83.06 mg, 0.5 mmol, 1.0 equivalent), sodium acetate (123.03 mg, 1.5 mmol, 3.0 equivalents), acetic anhydride (204.18 mg, 2.0 mmol, 4.0 equivalents), and dried DMF (5 mL) were added. The mixture was stirred in a nitrogen atmosphere at 170°C for 5 hours, whereupon the starting materials were almost dissolved. The heating was stopped and the mixture was cooled to room temperature.

The reaction liquid was poured into a 1% hydrochloric acid aqueous solution (10 mL), and extracted with ethyl acetate (40 mL×1). The resulting organic layer was washed with saturated brine (20 mL×2), and dried over sodium sulfate (5 g).

The solvent was evaporated under a reduced pressure, and the resulting residue was purified by silica gel chromatography [2.5 (diameter)×8 cm, 20 g; n-hexane:ethyl acetate (3:1); 150+50 mL)] to obtain methylenedioxycoumarin (64.3 mg, yield = 67.6%) as light brown crystals.
Melting point: 198-205°C (decomposition)
TLC: Rf = 0.27 (n-hexane/ethyl acetate = 2:1)
$^1$H-NMR ($CDCl_3$, δ ppm):6.07 (s, 6H, $Me_2$), 6.26 (d, J=9.76Hz, 1H), 6.82 (s, 2H), 7.56 (d, J=9.76Hz, 1H)
IR (KBr $cm^{-1}$): 3072, 2928, 1712, 1636, 1584, 1494, 1456, 1420, 1388, 1318, 1272, 1260, 1228, 1188, 1162, 1124, 1082, 1040, 942, 922, 884, 850, 838, 766, 752, 732, 552, 514

(2) Synthesis of esculetin

The reaction procedure of synthesis of esculetin is shown as follows. In the following reaction procedures, EtSH is ethanethiol.

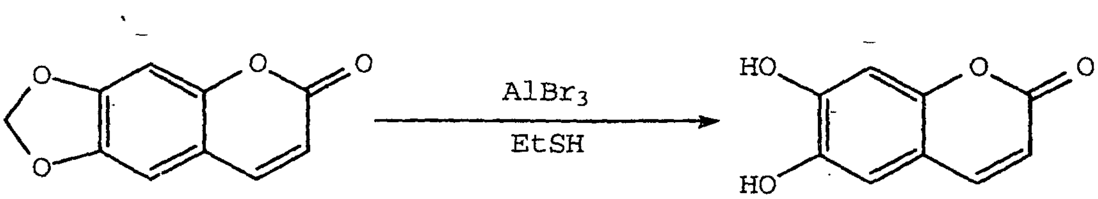


The actual procedures are as follows.

To an eggplant type flask (25 mL), ethanethiol (2 mL) and aluminum tribromide (320.9 mg, 1.2 mmol, 4.8 equivalents) were added and cooled to 0°C. While stirring, 6,7-methylenedioxycoumarin (47.5 mg, 0.25 mmol, 1.0 equivalent) was added in one portion. The reaction liquid was changed in color from pale brown to brown. The reaction liquid was stirred at 0°C for 2 hours, and then stirred in a draft at room temperature overnight to evaporate ethanethiol.

A 10% hydrochloric acid aqueous solution (10 mL) was added to the resulting residue to form a light brown precipitate. After stirring overnight, extraction with ethyl acetate (20 mL×2) was carried out. The resulting organic layer was washed with saturated brine (10 mL×2), and dried over sodium sulfate (5 g).

The solvent was evaporated under a reduced pressure, and the resulting residue (41.6 mg) was purified by silica gel chromatography [2.5(diameter)×1 cm, 2 g; ethyl acetate] to obtain esculetin (37.6 mg, yield = 84.4%) as light brown crystals. The IR spectrum of the compound accorded with that of the standard.
TLC: Rf = 0.57 ($CHCl_3$:MeOH:$CH_3COOH$:$H_2O$ = 16:8:1:2:)

IR (KBr $cm^{-1}$): 3232, 2364, 1672, 1626, 1564, 1524, 1460, 1404, 1282, 1200, 1148, 946, 878, 848, 820, 748, 670, 636, 576, 508, 454, 418, 552

Example 7

(1) Synthesis of esculetin acetonide

The reaction procedure of synthesis of esculetin acetonide is shown as follows.

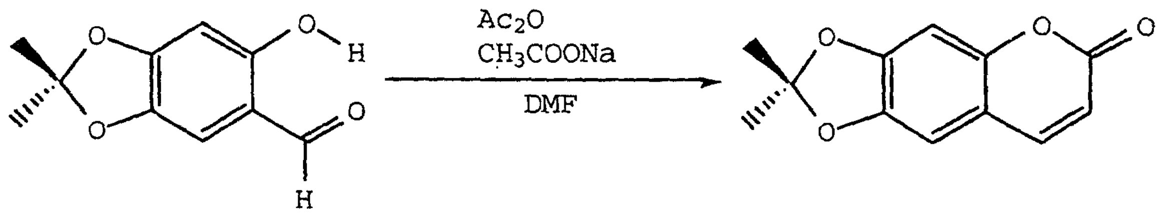


The actual procedures are as follows.

To an eggplant type flask (100 mL), 2,2-dimethyl-5-formyl-6-hydroxy-1,3-benzodioxole (164.3 mg, 0.85 mmol, 1.0 equivalent), sodium acetate (278 mg, 3.38 mmol, 4.0 equivalents), acetic anhydride (259 mg, 2.54 mmol, 3.0 equivalents), and dried DMF (10 mL) were added. The mixture was stirred in a nitrogen atmosphere at 170°C for 5 hours, whereupon the starting materials were almost dissolved. The heating was stopped and the mixture was cooled to room temperature.

The reaction liquid was poured into distilled water (40 mL) with floating ice, and extracted with ethyl acetate (40 mL×2). The resulting organic layer was washed with distilled water (20 mL×3) and saturated brine (20 mL×1), and dried over sodium sulfate (5 g).

The solvent was evaporated under a reduced pressure, and the resulting residue (200.2 mg) was purified by silica gel chromatography [2.5(diameter)×4 cm, 10 g; n-hexane:ethyl acetate (6:1); 180+30 mL)] to obtain esculetin acetonide (158.1 mg, yield = 85.7%) as light brown crystals.
Melting point: 178-180°C
TLC: Rf = 0.35 (n-hexane/ethyl acetate = 2:1)
$^1$H-NMR ($CDCl_3$, $\delta$ ppm): 1.72 (s, 6H, $Me_2$), 6.25 (d, J=9.76Hz, 1H), 6.74 (s, 1H), 6.75 (s, 1H), 7.57 (d, J=9.76Hz, 1H)
IR (KBr $cm^{-1}$): 3064, 3012, 2364, 1726, 1628, 1580, 1504, 1454, 1410, 1386, 1342, 1284, 1230, 1198, 1150, 1106, 1064, 982, 924, 890, 840, 824, 782, 754, 724, 650, 604, 558, 502, 464, 450

(2) Synthesis of esculetin

The reaction procedure of synthesis of esculetin is shown as follows.

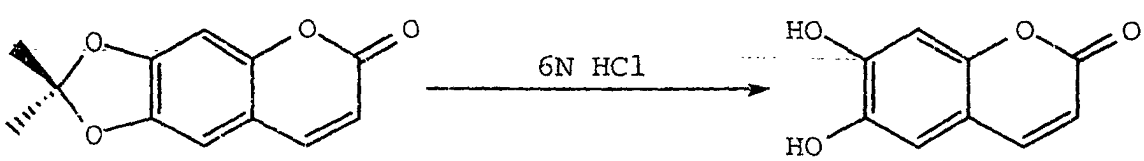


The actual procedures are as follows.

To an eggplant type flask (100 mL), esculetin acetonide (67.1 mg, 0.31 mmol, 1.0 equivalent) and a 6N hydrochloric acid aqueous solution (1 mL) were added. The mixture was stirred in a nitrogen atmosphere at 120°C for 2 hours, whereupon the starting materials were almost dissolved. The heating was stopped and the mixture was cooled to room temperature.

The reaction liquid was poured into distilled water (10 mL) with floating ice, and extracted with ethyl acetate (20 mL×3). The resulting organic layer was washed with distilled water (10 mL×3) and saturated brine (10 mL×1), and dried over sodium sulfate (5 g). The solvent was evaporated under a reduced pressure, and the resulting residue was purified by silica gel chromatography [1.7(diameter)×5 cm, 4 g; ethyl acetate] to obtain esculetin (39.4 mg, yield

= 72.0%) as light brown crystals.
TLC: Rf = 0.57 ($CHCl_3$:MeOH:$CH_3COOH$:$H_2O$ = 16:8:1:2)
IR (KBr $cm^{-1}$): 3352, 1671, 1623, 1569, 1404, 1368, 1284, 1149, 948, 879, 849, 822, 672, 636, 579

When deprotection reaction was attempted using an acetic acid: distilled water (4:1) mixture instead of the 6N hydrochloric acid aqueous solution, the reaction rate thereof was slow. However, after the reaction was completed, the resulting residue was purified by silica gel chromatography [1.7 (diameter) ×5 cm, 4 g; ethyl acetate] to obtain esculetin (62.2 mg, yield = 83.7%) as light brown crystals.
TLC: Rf = 0.57 ($CHCl_3$:MeOH:$CH_3COOH$:$H_2O$ = 16:8:1:2)
IR (KBr $cm^{-1}$): 2364, 1672, 1626, 1572, 1404, 1366, 1284, 1200, 1150, 948, 878, 848, 822, 670, 636, 578

[Synthetic examples of aldehyde compounds as a starting material]

Synthetic example 1: 2,5-dihydroxy-4-methoxybenzaldehyde (the starting material in Example 2)

The reaction procedure of the present synthetic example is shown as follows.

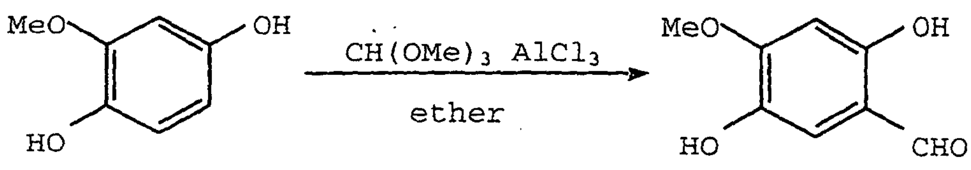


The actual procedures are as follows.

Trimethyl orthoformate (2.89 mL, 6.0 equivalents) was added dropwise to methoxyhydroquinone (618 mg, 4.41 mmol), and then ether (15.5 mL) was further added. After methoxyhydroquinone was completely dissolved, the reaction system was placed in a water bath, and aluminum chloride (882 mg, 1.5 equivalents) was added in a nitrogen gas stream under stirring. After the reaction was carried out for 10 minutes, a 10% hydrochloric acid (50 mL) was added, and then extraction with ether (30 mL×3) and washing with water (50 mL×3) and saturated brine (50 mL×3) were carried out. The resulting organic layer was dried over sodium sulfate, and concentrated under a reduced pressure to obtain 2,5-dihydroxy-4-methoxybenzaldehyde (166 mg, yield = 24.5%) as light yellow crystals.
$^1$H-NMR ($CD_3OD$): δ 3.85 (3H, s, $CH_3O$-), 6.44 (1H, s, H-3), 6.94 (1H, s, H-6), 9.66 (1H, s, -CHO)

Synthetic example 2:Synthesis of 4-benzyloxy-5-hydroxysalicylaldehyde (the starting material in Example 3)

The reaction procedure of the present synthetic example is shown as follows. In the following reaction procedures, MEK is methyl ethyl ketone.

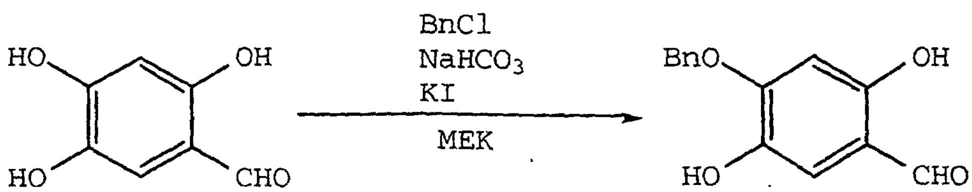


The actual procedures are as follows.

To an eggplant type flask (50 mL), 2,4,5-trihydroxybenzaldehyde (154 mg, 1.0 mmol, 1.0 equivalent), sodium hydrogencarbonate (126 mg, 1.5 mmol, 1.5 equivalents), potassium iodide (33 mg, 0.2 mmol, 0.2 equivalent), and methyl ethyl ketone (MEK; 10 mL) were added. To this solution, benzyl chloride (189.9 mg, 1.5 mmol, 1.5 equivalents) was added portionwise at room temperature.

The reaction liquid was heated at reflux in a nitrogen atmosphere at 105°C for 22 hours in total. The starting materials appeared to be completely dissolved, and thus the reaction liquid was evaporated under a reduced pressure.

An acid aqueous solution (10 mL, a 1% hydrochloric acid aqueous solution) was poured, and extraction with

ethyl acetate (30 mL×1) was carried out. An organic layer was washed with saturated brine (10 mL×1) distilled water (10 mL×1), and further saturated brine(10 mL×1), dried over sodium sulfate (5 g), and concentrated under a reduced pressure to obtain a crude product (297.8 mg). The crude product was purified by silica gel chromatography [1.7 (diameter) ×8 cm, 6 g; n-hexane:ethyl acetate (4:1)] to obtain 4-benzyloxy-5-hydroxysalicylaldehyde (219.1 mg, yield = 89.7%) as pale brown crystals.
Melting point: 144-145°C
TLC: Rf = 0.5 (n-hexane/ethyl acetate = 2:1)
$^1$H-NMR ($CDCl_3$, δ ppm): 5.15 (s, 2H, $PhCH_2$), 6.54 (s, 1H, Ar-H), 7.04 (s, 1H, Ar-H), 7.42 (s, 5H, Ph), 9.67 (s, 1H, CHO)
IR (KBr $cm^{-1}$): 3332, 1638, 1588, 1560, 1504, 1470, 1398, 1362, 1218, 1178, 1142, 990, 914, 866, 834, 798, 746, 698, 602, 552, 482, 410

Synthetic example 3: Synthesis of 2,5-dihydroxy- 4-benzyloxybenzaldehyde (the starting material in Example 3)

(1) Synthesis of 2-benzyloxy-1,4-benzoquinone 1

The reaction procedure of synthesis of 2-benzyloxy-1,4-benzoquinone is shown as follows.

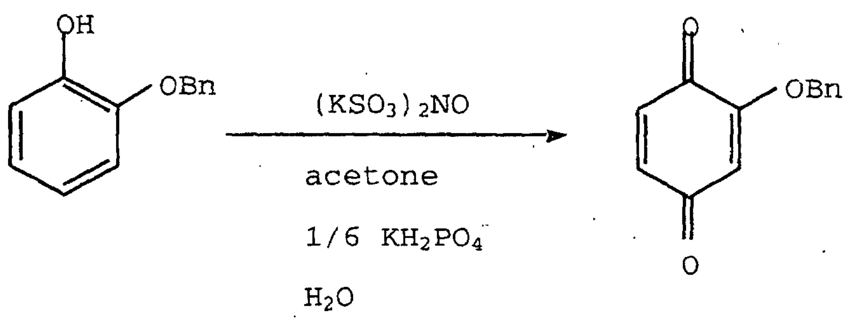


The actual procedures are as follows.
To an eggplant type flask (100 mL), 2-(benzyloxy)phenol (200.2 mg, 1.0 mmol, 1.0 equivalent) and acetone (5 mL) were added. To this stirred solution, distilled water (15 mL) containing $KH_2PO_4$ (204 mg, 1.5 mmol, 1.5 equivalents) was added. A Fremy salt (i.e., potassium nitrososulfonate; 1.07 g, 4.0 mmol, 4.0 equivalents) was dissolved in distilled water (40 mL) cooled to 4°C and containing $KH_2PO_4$ (544 mg, 4.0 mmol, 4.0 equivalents), and the solution was quickly added dropwise at room temperature. After the dropping was completed, the mixture was stirred at room temperature for 3 hours, and then the solution color was changed to dark brown and a yellow precipitate was formed. It was observed that the starting materials were almost dissolved at this stage.
After the reaction was completed, ethyl acetate (40 mL×1, 20 mL×1) was added, and an aqueous layer was extracted. The resulting organic layer was washed with saturated brine (10 mL×1), dried over sodium sulfate (5 g), and concentrated under a reduced pressure to obtain crude solid crystals (224.3 mg). The crude crystals were purified by silica gel chromatography [21.7 (diameter) ×7.5 cm, 6 g; n-hexane:ethyl acetate (6:1)] to obtain 2-benzyloxy-1,4-benzoquinone (182.9 mg, yield = 83.4%) as pale brown crystals.
TLC: Rf = 0.41 (n-hexane/ethyl acetate = 2:1
$^1$H-NMR ($CDCl_3$, δ ppm): 5.05 (s, 2H, $CH_2Ph$), 6.00 (s, 1H, H-3), 6.71 (s, 2H, H-5, 6), 7.40 (s, 5H, Ph-)
IR (KBr $cm^{-1}$): 3080, 2344, 1674, 1642, 1596, 1504, 1454, 1386, 1360, 1316, 1248, 1210, 1112, 1004, 886, 836, 728, 692, 466, 424

(2) Synthesis of 2-benzyloxy-1,4-benzoquinone 2

The reaction procedure of synthesis of 2-(benzyloxy)hydroquinone is shown as follows.

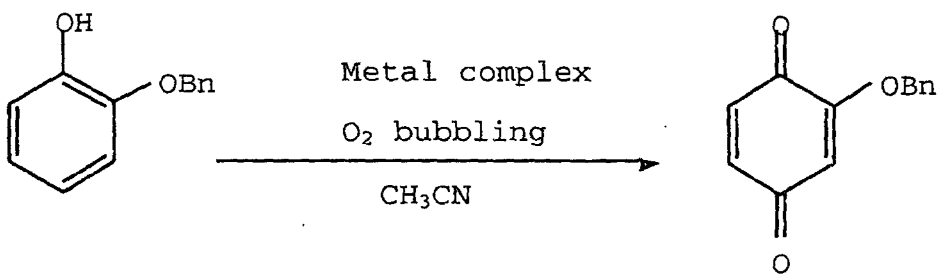


The actual procedures are as follows.

To an eggplant type flask (100 mL), 2-benzyloxyphenol (206 mg, 1.03 mmol, 1.0 equivalent) and acetonitrile (20 mL) were added. To this stirred solution, Co(bpb)$H_2O$ (38.5 mg, 0.098 mmol, 0.05 equivalent) was added at room temperature. The mixture color was changed from brownish green to brown. In this connection, Co(bpb)$H_2O$ is aqua-[N, N-bis( (2'-pyridine-carboxamido)-1,2-benzene]cobalt (II).

Oxygen was blown into the reaction mixture at the flow rate of 100 mL/min for 24 hours in total, but the starting materials still remained. After the reaction was completed, the solvent was concentrated under a reduced pressure and evaporated. The resulting residue was purified by silica gel chromatography [2.5(diameter)×4.0 cm, 10 g; n-hexane:ethyl acetate (10:1)] to obtain 2-benzyloxy-1,4-benzoquinone (93.5 mg, yield = 42.4%) in Fraction Nos. 11-16 as golden crystals, and further obtain the starting material (114.7 mg, 55.6%) in Fraction Nos. 2-4 as colorless oil. This yield corresponds to 95.5% in view of the recovery of the starting material.
TLC: Rf = 0.41 (n-hexane/ethyl acetate = 2:1)

(3) Synthesis of 1,4-diacetoxy-2-benzyloxybenzene

The reaction procedure of synthesis of 1,4-diacetoxy-2-benzyloxybenzene is shown as follows.

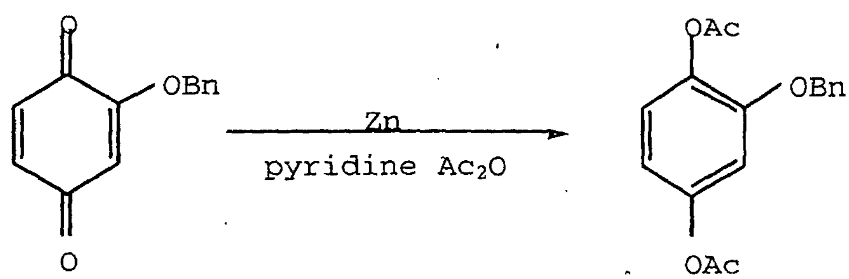


The actual procedures are as follows.

To an eggplant type flask (25 mL), 2-benzyloxy-1,4-benzoquinone (250 mg, 1.17 mmol, 1.0 equivalent), acetic anhydride (2.5 mL, 227.0 equivalents), and zinc powder (250 mg, 3.82 mmol, 3.28 equivalents) were added at. 0°C. Pyridine (0.25 mL, 3.69 mmol, 2.64 equivalents) was added portionwise to the solution at 0°C.

After 20 minutes, the reaction liquid color was changed from a brown precipitated state to light green, and further stirred for 45 minutes.

The reaction mixture was filtered through a glass filter (3G4) to remove zinc powder and inorganic salts, and the residue was sufficiently washed ethyl acetate. The resulting filtrate was diluted with ethyl acetate, and washed with saturated sodium hydrogencarbonate (5 mL).

The organic layer was washed with distilled water (5 mL×2) and saturated brine (5 mL×1), dried over sodium sulfate (5 g), and concentrated under a reduced pressure to obtain a crude product (438 mg). The crude product was purified by silica gel chromatography [2.5 (diameter) ×3 cm, 8 g; n-hexane:ethyl acetate (3:1)] to obtain 1,4-diacetoxy-2-benzyloxybenzene (344.5 mg, yield = 98.3%) as pale brown crystals.
TLC: Rf = 0.41 (n-hexane/ethyl acetate = 2:1)
$^1$H-NMR ($CDCl_3$, δ ppm): 2.25 (s, 3H, $COCH_3$), 2.27 (s, 1H, $COCH_3$), 5.05 (s, 2H, $PhCH_2$), 6.70 (dd, J=8.30, 2.44Hz, 1H, Ar-H-6), 6.78 (d, J=2.44Hz, 1H, Ar-H-5), 7.04 (d, J-8.30Hz, 1H, Ar-H-3)
IR (KBr $cm^{-1}$): 3092, 3048, 1766, 1620, 1598, 1510, 1478, 1458, 1428, 1392, 1374, 1282, 1222, 1184, 1152, 1112,

1048, 1010, 972, 914, 888, 840, 826, 766, 742, 720, 700, 634, 624, 602, 588, 484, 468

(4) Synthesis of 2-benzyloxy-1,4-hydroquinone

The reaction procedure of synthesis of 2-benzyloxy-1,4-hydroquinone is shown as follows.

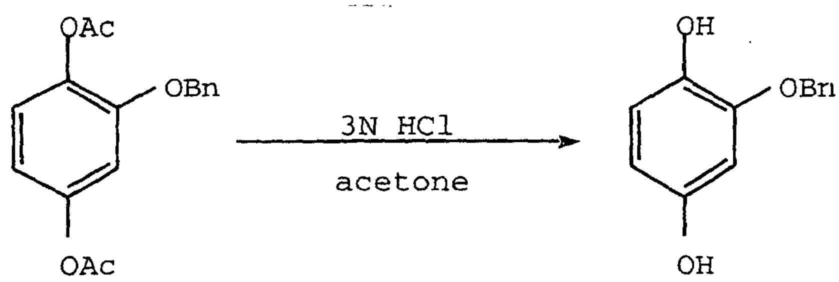


The actual procedures are as follows.

To an eggplant type flask (10 mL), 1,4-diacetoxy-2-benzyloxybenzene (35.2 mg, 1.17 mmol, 1.0 equivalent) and acetone (1 mL) were added. A 2% hydrochloric acid aqueous solution (0.5 mL) was added to this stirred solution.

The mixture was heated under reflux for 4 hours.

After the reaction was completed, the solvent was removed under a reduced pressure to obtain a crude product (24 mg). The crude product was purified by silica gel chromatography [1.7 (diameter) ×6.5 cm, 5 g; n-hexane: ethyl acetate (2:1, 100 mL + 50 mL)] to obtain 2-benzyloxy-1,4-hydroquinone (18.2 mg, yield = 71.8%) as colorless crystals.
Melting point: 99-101°C
TLC: Rf = 0.24 (n-hexane/ethyl acetate = 2:1)
$^1$H-NMR ($CDCl_3$, δ ppm): 5.02 (s, 2H, $CH_2$Ph), 5.32 (br, 1H, OH, disappearance by adding heavy water), 6.31 (dd, J=8.79, 2.93Hz, 1H, H-6), 6.50 (d, J=8 2.93Hz, 1H, H-3), 6.78 (d, J=8.79Hz, 1H, H-5), 7.40 (s, 5H, Ph-)
IR (KBr $cm^{-1}$): 3492, 3412, 1632, 1512, 1484, 1386, 1356, 1320, 1296, 1258, 1224, 1160, 1114, 1026, 946, 832, 792, 752, 722, 706, 628, 448

| Elemental analysis: | |
|---|---|
| Calculated: | C 72.21, H 5.59 |
| Found: | C 72.0, H 5.5 |

(5) Synthesis of 2,5-dihydroxy-4-benzyloxybenzaldehyde (the starting material in Example 3)

The reaction procedure of synthesis of 2,5-dihydroxy-4-benzyloxybenzaldehyde is shown as follows.

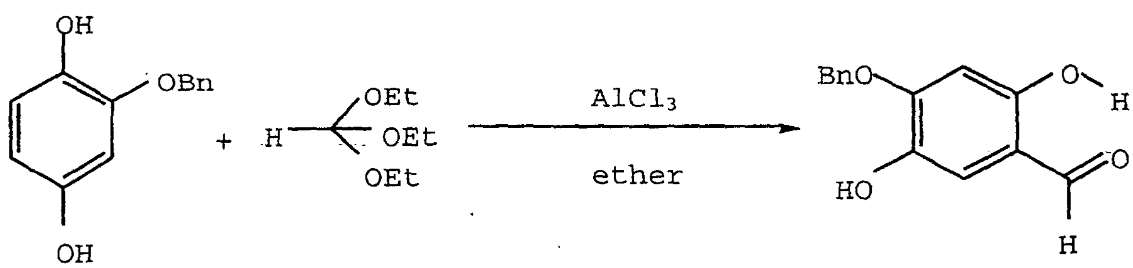


The actual procedures are as follows.

To an eggplant type flask (25 mL), 2-benzyloxyhydroquinone (108 mg, 0.5 mmol, 1.0 equivalent), triethyl orthoformate (600 mg, 4.04 mmol, 8 equivalents), and diethyl ether (3 mL) were added. The mixture was cooled to 8°C, and aluminum chloride (100 mg, 0.75 mmol, 1.5 equivalents) was added portionwise. The reaction liquid color

was changed from light green to dark green immediately. The reaction was carried out for 6 minutes in this state.

The starting materials were completely dissolved, and thus the reaction liquid was cooled to 0°C. An acid aqueous solution (10 mL) was added to the solution, and the whole was extracted with ether (40 mL×1, 20 mL×1) and ethyl acetate (20 mL×2). The resulting organic layer was washed with distilled water (10 mL×1) and saturated brine (10 mL×1), dried over sodium sulfate (5 g), and concentrated under a reduced pressure to obtain a crude product as a solid.

The crude product was purified by silica gel chromatography [1.7(diameter)×6.5 cm, 5 g; n-hexane:ethyl acetate (5:1)] to obtain 2,5-dihydroxy-4-benzyloxybenzaldehyde (76.2 mg, yield = 63.2%) as light brown crystals.
Melting point: 147-148°C
TLC: Rf = 0.44 (n-hexane/EtOH = 2:1)
$^1$H-NMR ($CDCl_3$, δ ppm): 5.15 (s, 2H, $PhCH_2$), 6.54 (s, 1H, Ar-H), 7.04 (s, 1H, Ar-H), 7.42 (s, 5H, Ph), 9.67 (s, 1H, CHO)
IR (KBr $cm^{-1}$): 3332, 1638, 1588, 1560, 1504, 1470, 1398, 1362, 1218, 1178, 1142, 990, 914, 866, 834, 798, 746, 698, 602, 552, 482, 410

| Elemental analysis: | |
|---|---|
| Calculated: | C 68.85, H 4.95 |
| Found: | C 69.0, H 5.1 |

Synthetic example 4: Synthesis of 2,4,5-triacetoxybenzaldehyde (the starting material in Example 4)

The reaction procedure of the present synthetic example is shown as follows.

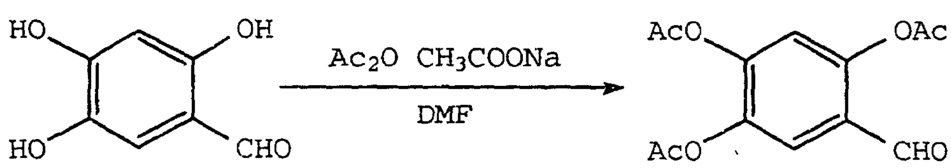


The actual procedures are as follows.

Acetic anhydride (0.19 mL, 4.0 equivalents) was added dropwise to a solution of sodium acetate (80.7 mg, 6.0 equivalents) in DMF (0.98 mL), and stirred at room temperature for 15 minutes. A solution of 2,4,5-trihydroxybenzaldehyde (75.8 mg, 0.492 mmol) in DMF (0.30 mL×3) was added dropwise to the reaction system, and the reaction was carried out at room temperature for 20 minutes. After dissolution of the starting materials was confirmed by TLC, a 10% hydrochloric acid aqueous solution (30 mL) was added under stirring while cooling on ice to precipitate white crystals. The crystals were filtered through a KIRIYAMA ROHTO and washed with water to obtain 2,4,5-triacetoxybenzaldehyde (135 mg, 97%) as white crystals.

Synthetic example 5: Synthesis of 2,5-diacetoxy-4-benzyloxybenzaldehyde (the starting material in Example 5)

The reaction procedure of the present synthetic example is shown as follows.

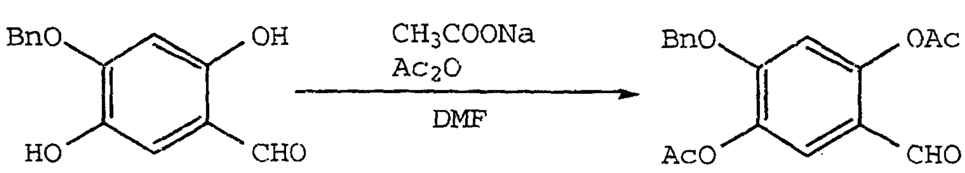


The actual procedures are as follows.

To an eggplant type flask (100 mL), 4-benzyloxy-2,5-dihydroxyaldehyde (193.8 mg, 0.793 mmol, 1.0 equivalent), sodium acetate (82.05 mg, 1.0 mmol, 1.23 equivalents), and dried DMF (1 mL) were added. An acetic anhydride (204.2 mg, 2.0 mmol, 2.5 equivalents) was added portionwise to the solution at room temperature.

The reaction liquid was stirred in a nitrogen atmosphere at room temperature for 2 hours, whereupon the

starting materials were completely dissolved. Water (10 mL) was poured to form a white precipitate, and the precipitate was dissolved by adding ether (20 mL) and ethyl acetate (10 mL). The resulting organic layer was washed with water (10 mL×3), dried over sodium sulfate (5 g), and concentrated under a reduced pressure to obtain a crude product. The crude product was washed with n-hexane to obtain 2,5-diacetoxy-4-benzyloxybenzaldehyde (236.8 mg, yield = 90.89%) as pale cream crystals.
Melting point: 136-137°C
TLC: Rf = 0.28 (n-hexane/ethyl acetate = 2:1)
$^1$H-NMR ($CDCl_3$, δ ppm): 2.27 (s, 3H, $CH_3CO$), 2.38 (s, 3H, $CH_3CO$), 5.13 (s, 2H, $PhCH_2$), 6.81 (s, 1H, Ar-H), 7.38 (s, 5H, Ph), 7.58 (s, 1H, Ar-H), 9.95 (s, 1H, CHO)
IR (KBr $cm^{-1}$): 2340, 1774, 1690, 1618, 1510, 1424, 1370, 1328, 1284, 1216, 1156, 1102, 1008, 944, 830, 786, 770, 742, 700, 668, 616, 596, 484

| Elemental analysis: | |
|---|---|
| Calculated: | C 65.85, H 4.91 |
| Found: | C 66.2, H 5.0 |

Synthetic example 6: Synthesis of 4,5-methylenedioxysalicylaldehyde (the starting material in Example 6)

The reaction procedure of the present synthetic example is shown as follows.

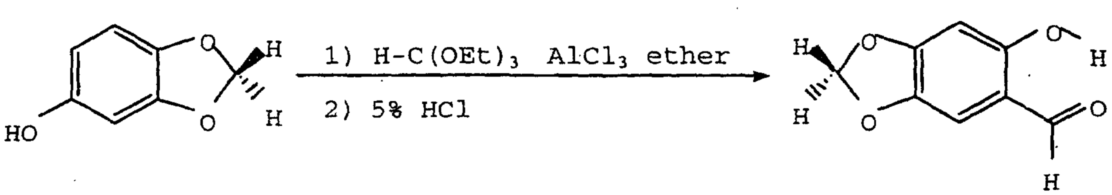


The actual procedures are as follows.

To an eggplant type flask (100 mL), sesamol (1.38 g, 10.0 mmol, 1.0 equivalent) and ether (40 mL) were added and dissolved. Triethyl orthoformate (10 mL, 7.6 mmol, 7.6 equivalents) was added to the solution. To the resulting yellow solution, aluminum chloride (2.0 g, 15 mmol, 1.5 equivalents) was added portionwise at 0°C. The mixture color was changed from light red to green, and further, from dark green to dark blue. After 5 minutes, the mixture was cooled to 0°C, and a 5% hydrochloric acid solution (10 mL) was added. The resulting product was slightly soluble, and the separation thereof was difficult.

The separation was carried out by adding distilled water (1 0mL) and ethyl acetate (40 mL). The aqueous layer was further extracted with ethyl acetate (40 mL). The collected organic layer was washed with saturated brine (10 mL), dried over sodium sulfate (approximately 5 g), and concentrated and solidified by an evaporator to obtain a dark red solid (1.6 g).

From the resulting dark red solid, 506.6 mg thereof was taken and purified by silica gel chromatography [2.5 (diameter) ×8.0 cm, 20 g; n-hexane:ethyl acetate (3:1)] to obtain 4,5-methylenedioxysalicylaldehyde (363.9 mg, yield = 70.9%) in Fraction Nos. 2-4 as light brown needle crystals.
Melting point: 125-126°C
TLC: Rf = 0.63 (n-hexane/ethyl acetate = 2:1)
$^1$H-NMR ($CDCl_3$, δ ppm): 6.02 (s, 6H, $Me_2$), 6.47 (s, 1H), 6.86 (s, 1H), 9.62 (s, 1H, CHO)
IR (KBr $cm^{-1}$): 3076, 1646, 1614, 1506, 1482, 1424, 1388, 1314, 1270, 1230, 1162, 1086, 1036, 934, 872, 854, 786, 774, 722, 710, 514

Synthetic example 7

(1) Synthesis of 2,2-dimethyl-1,3-benzodioxol

The reaction procedure of synthesis of 2,2-dimethyl-1,3-benzodioxol is shown as follows. In the following reaction procedures, TsOH is p-toluenesulfonic acid.

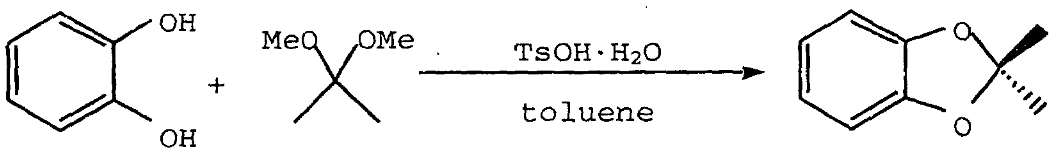


The actual procedures are as follows.

To an eggplant type flask (50 mL), catechol (605 mg, 5.5 mmol, 1.1 equivalents), 2,2-dimethoxypropane (520 mg, 5.0 mmol, 1.0 equivalent), p-toluenesulfonic acid (approximately 2 mg), and toluene (40 mL) were added. The mixture was distilled at 130°C for 6 hours by azeotropic distillation, but the starting materials still remained. The heating was stopped and the mixture was cooled to room temperature. A drop of a 28% sodium methylate in methanol solution was added to neutralize the mixture.

The solvent was removed under a reduced pressure. The resulting residue (689.9 mg) was purified by silica gel chromatography [2.5 (diameter) ×4 cm, 10 g; n-hexane:ethyl acetate (4:1); 160+40 mL] to obtain 2,2-dimethyl-1,3-benzodioxol (373 mg, yield = 49.6%) as yellow oil, and the starting material (216 mg, 35.8%) at the same time.
TLC: Rf = 0.91 (n-hexane/ethyl acetate = 2:1)
$^1$H-NMR ($CDCl_3$, δ ppm): 1.67 (s, 6H, $Me_2$), 6.75 (m, 4H)
IR (KBr $cm^{-1}$): 3080, 3004, 2948, 1632, 1490, 1382, 1370, 1240, 1112, 1006, 978, 908, 840, 820, 786, 738, 434

(2) Synthesis of 5-hydroxy-2,2-dimethyl-1,3-benzodioxol

The reaction procedure of synthesis of 5-hydroxy-2,2-dimethyl-1,3-benzodioxol is shown as follows.

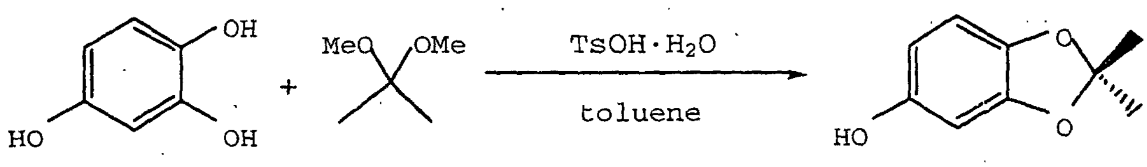


The actual procedures are as follows.

To an eggplant type flask (50 mL), 1,2,4-trihydroxybenzene (630.6 mg, 5.0 mmol, 1.0 equivalent), 2,2-dimethoxypropane (781 mg, 7.5 mmol, 1.5 equivalents), p-toluenesulfonic acid (approximately 1 mg), and toluene (40 mL) were added. The mixture was distilled at 130°C for 1.5 hours by azeotropic distillation, but the starting materials still remained. The heating was stopped and the mixture was cooled to room temperature. A drop of a 28% sodium methylate in methanol solution was added to neutralize the mixture.

The solvent was removed under a reduced pressure. The resulting residue (831.5mg) was purified by silica gel chromatography [2.5(diameter)×4 cm, 10 g; n-hexane:ethyl acetate (4:1); 160+40 mL] to obtain 5-hydroxy-2,2-dimethyl-1,3-benzodioxol (240.9 mg, yield = 28.9%) as light brown oil, and further a by-product (45.9 mg).
TLC: Rf = 0.47 (n-hexane/ethyl acetate = 2:1)
$^1$H-NMR ($CDCl_3$, δ ppm): 1.65 (s, 6H, , $Me_2$), 6.20 (dd, J=8.30, 2.45Hz, 1H), 6.35 (d, J=2.45Hz, 1H), 6.55 (d, J=8.30Hz, 1H)
IR (KBr $cm^{-1}$): 3396, 3004, 1626, 1498, 1382, 1224, 1160, 1118, 1074, 980, 948, 836, 788, 764, 610, 424, 884

(3) Synthesis of 2,2-dimethyl-5-formyl-6-hydroxy-1,3-benzodioxol (the starting material in Example 7)

The reaction procedure of synthesis of 2,2-dimethyl-5-formyl-6-hydroxy-1,3-benzodioxol is shown as follows.

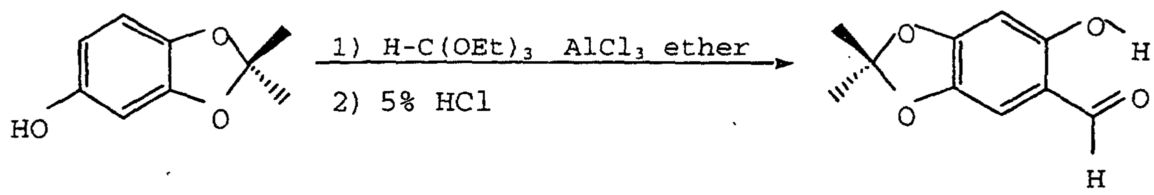


The actual procedures are as follows.

To an eggplant type flask (100 mL), 5-hydroxy-2,2-dimethyl-1,3-benzodioxol (240 mg, 1.45 mmol, 1.0 equivalent) and ether (5 mL) were added and dissolved. Triethyl orthoformate ester (1.5 mL, 7.6 mmol, 7.6 equivalents) was added to the solution. To the resulting yellow solution, aluminum chloride (289 mg, 2.17 mmol, 1.0 equivalent) was added portionwise at 0°C. The mixture color was changed from light red to green, and further, from dark green to dark blue. After 5 minutes, the mixture was cooled to 0°C, and a 2% hydrochloric acid solution (10 mL) was added.

Ether (20 mL) was added and extraction was carried out. The collected organic layer was washed with distilled water (10 mL×2), dried over sodium sulfate (approximately 3 g), and concentrated and solidified by an evaporator to obtain a dark red solid (187.3 mg). The resulting solid was purified by silica gel chromatography [1.7 (diameter) ×6.0 cm, 5 g; n-hexane:ethyl acetate (10:1); 150 mL + 15 mL] to obtain a fraction (106 mg, 37.6%) containing the desired product in Fraction Nos. 1-3.

Further, the fraction was purified by silica gel chromatography [1.7(diameter)×8.5 cm, 8 g; n-hexane:ethyl acetate (10:1); 100 mL + 10 mL] to obtain 2,2-dimethyl-5-formyl-6-hydroxy-1,3-benzodioxol (yield = 37.6%) in Fraction Nos. 1-3 as light yellow needle crystals.
Melting point:104-105°C
TLC: Rf = 0.68 (n-hexane/ethyl acetate = 2:1)
$^1$H-NMR ($CDCl_3$, δ ppm): 1.69 (s, 6H, $Me_2$), 6.37 (s, 1H, H-7), 6.76 (s, 1H, H-4), 9.60 (s, 1H, CHO)
IR (KBr $cm^{-1}$): 3008, 1650, 1614, 1494, 1422, 1394, 1388, 1382, 1340, 1282, 1248, 1182, 1156, 1068, 984, 882, 844, 800, 782, 758, 704, 524, 470, 430

Assay example 1: Assay for antifungal activity of intermediates of esculetin compounds

(1) Compounds to be tested

Antifungal activities of the following Compounds (a) to (e) were examined.

Compound (a): 2-benzyloxy-1,4-hydroquinone [the compound in Synthesis example 3(4)]
Compound (b): 2,5-dihydroxy-4-benzyloxybenzaldehyde [the compound in Synthesis example 3(5)]
Compound (c): 2,5-diacetoxy-4-benzyloxybenzaldehyde [the compound in Synthesis example 5]
Compound (d): 4,5-methylenedioxysalicylaldehyde [the compound in Synthesis example 6]
Compound (e): 2,2-dimethyl-5-formyl-6-hydroxy-1,3-benzodioxol [the compound in Synthesis example 7(3)]

(2) Pathogenic fungi to be tested

Antifungal activities against the following five plant pathogenic fungi were examined.

(i) Pyricularia oryzae(P.o in Table 1)
(ii) Fusarium nivale (F.niv in Table 1)
(iii) Septoria tritici(S.t in Table 1)
(iv) Pythium aphanidermatum (P.a in Table 1)
(v) Pytophthora infestans (P.i in Table 1)

(3) Procedures in assay

Each of the test compounds (a) to (e) was dissolved in dimethylformamide at the concentration of 1 mg/mL to prepare each solution for assay.

In Pyricularia oryzae (P.o) and Pythium aphanidermatum (P.a) among the above plant pathogenic fungi, distilled water (10 mL) was added to a petri dish in which spores were formed, and the concentration of spores was adjusted to 1×$10^5$ spores/mL. In Pytophthora infestans (P.i), distilled water (10 mL) was added to a petri dish in which

spores were formed, and the concentration of spores was adjusted to $1\times10^4$ spores/mL.

In Fusarium nivale (F.niv) and Septoria tritici(S.t), a culture in a PDA medium (potato, dextrose, and agar medium) was carried out until the fungus was fully grown. The colony taken from one petri dish was transferred with the medium to a vessel of a homogenizer. Sterile water (50 mL) was added to the vessel, and the whole was dispersed by the homogenizer at 5000 rpm for 30 seconds while cooling, and then filtered through two sheets of sterile gauze. After centrifugation (3000 rpm, 5minutes), a pellet was suspended in sterile water (50 mL) (1 to 3 spores in a field of microscope at a magnification of $\times100$).

A suspension of spores or mycelia (10 mL) was added to a PD medium (potato and dextrose medium) (90 mL), and stirred well. In Pytophthora infestans (P.i), a rye liquid medium was used instead of the PD medium.

A flat-bottomed 96-well microplate was prepared. Each assay solution (1 μL) was added to the microplate, and then each medium (100 μL) containing an inoculum of each plant pathogenic fungus was further added, and stirred well. In a control assay of a group without a drug (i.e., not containing a test compound), only dimethylformamide (1 μL) was added. Three wells per each test compound were used.

In a control assay in which no plant pathogenic fungus was inoculated, an assay solution for control without inoculation was prepared by adding sterile water (10 mL) to a non-inoculated medium (90 mL), and the assay solution for control without inoculation (100 μL per a well of the microplate) was added.

A static culture was carried out at 20°C or 25°C.

(4) Method for evaluation and results

The culture was carried out for a predetermined period (DAT) described in the following Table 1, and then $OD_{595}$ was measured using a microplate reader. A rate of suppression was calculated in accordance with the following equation (1):

$$[1-(T-R)/(C-R)]\times100 \qquad (1)$$

wherein T is an absorbance ($OD_{595}$) in an assay group in which an assay solution containing a test compound was added to a medium containing an inoculum of a plant pathogenic fungus; C is an absorbance ($OD_{595}$) in a non-drug group in which an assay solution without a test compound was added to a medium containing an inoculum of a plant pathogenic fungus; and R is an absorbance ($OD_{595}$) in a non-inoculated control assay group in which an assay solution without a test compound was added to a medium without an inoculum of a plant pathogenic fungus.

The rate of suppression calculated as above was evaluated as three grades in accordance with the following basis:

-: less than 10%
+: 10 to 60%
++: more than 60%

The results are shown in Table 1.

Table 1

| Test compound | Rate of growth suppression (%) | | | | |
|---|---|---|---|---|---|
| | P.o<br>3 DAT<br>10 mg/L | F.niv<br>4 DAT<br>10 mg/L | S.t<br>7 DAT<br>10 mg/L | P.a<br>1 DAT<br>10 mg/L | P.i<br>4 DAT<br>10 mg/L |
| Compound (a) | ++ | + | + | + | + |
| Compound (b) | + | ++ | + | ++ | + |
| Compound (c) | + | ++ | ++ | + | + |
| Compound (d) | + | + | + | + | + |
| Compound (e) | + | + | + | + | + |
| DAT: Days after treatment | | | | | |

Assay example 2: Assay for herbicidal activity of intermediates of esculetin compounds

(1) Compounds to be tested

Herbicidal activities of the above Compounds (a), (b), (d), and (e) used in the above assay example for the antifungal activity were examined.

(2) Plants to be tested

Effects of a germination suppression against the following four plants were examined:

(i) Amaranthus retroflexuso (Amaranthaceae)
(ii) Lactuca sativa (Asteraceae)
(iii) Solanum nigrum (Solanaceae)
(iv) Setaria viridis (Poaceae)

(3) Procedures in assay

Seeds of each test plant were treated with a 1% sodium hypochlorite solution for 2 minutes, washed with sterile water, and air-dried, and then the assay was carried out using the resulting seeds. A filter paper (Kiriyama; for a funnel; diameter = 21mm) was placed on a 12-well microplate, and seeds were seeded thereon.
Each test compound was dissolved in acetone at the concentration of 10 mg/mL, and diluted with a 2-fold dilution of Murashige-Skoog salts (MS) to 200-fold. The prepared test compound solution (0.3 mL) was added to the microplate, the edge thereof was covered with a cellophane tape, and the microplate was incubated in a temperature controlled room. In a control assay of a group without a drug (i.e., not containing a test compound), only a diluted liquid (0.3 mL) prepared by diluting acetone with a 2-fold dilution of Murashige-Skoog salts to 200-fold was added.

(4) Method for examination

Growth at 25°C under 4000 to 5000 lx (24 hours light condition) for 7 days was carried out, and then effects against Shoots and Roots were evaluated by a visual assessment. The evaluation was carried out as the following six grades:

0: same as that without treatment
1: suppressed by less than 20%
2: suppressed by a range from 20% to less than 40%
3: suppressed by a range from 40% to less than 60%
4: suppressed by a range from 60% to less than 80%
5: suppressed by 80% or more

The results are shown in Table 2. In Table 2, G is a germination inhibition, Ig is a growth suppression, Ir is a growth suppression of Roots, and Ur is negative geotropism.

Table 2

| Test compound | Concentration mg/L | Herbicidal activity (0 to 5) | | | | | | | |
|---|---|---|---|---|---|---|---|---|---|
| | | Amaranthus retroflexuso | | Lactuca sativa | | Solanum nigrum | | Setaria viridis | |
| Acetone | 5000 | 0 | | 0 | | 0 | | 0 | |
| Compound (a) | 50 | 5 | G | 4.8 | G | 5 | G | 1 | IglrUr |
| Compound (b) | 50 | 5 | G | 0 | | 5 | G | 3 | GlglrUr |
| Compound (d) | 50 | 0 | | 2 | Ir | 4 | Iglr | 0 | |
| Compound (e) | 50 | 0 | | 0 | | 4 | Iglr | 0 | |

Assay example 3: Assay for antifungal activity of esculetin compounds

The activities of the following esculetin compounds was confirmed by a method similar to Assay example 1.

7-benzyloxy-6-hydroxycoumarin [the compound in Example 3]
methylenedioxycoumarin [the compound in Example 6(1)]
esculetin acetonide [the compound in Example 7(1)]

Assay example 4: Assay for herbicidal activity of esculetin compounds

The activities of the following esculetin compounds was confirmed by a method similar to Assay example 2.

7-benzyloxy-6-hydroxycoumarin [the compound in Example 3]
methylenedioxycoumarin [the compound in Example 6(1)]
esculetin acetonide [the compound in Example 7(1)]

Preparative example 1

Powder was prepared from the following composition.

| | Parts by weight |
|---|---|
| Compound (a) in Assay example 1 | 3 |
| clay | 40 |
| talc | 57 |

The above components were ground and mixed to prepare powder.

Preparative example 2

Water dispesible powder was prepared from the following composition.

| | Parts by weight |
|---|---|
| Compound (b) in Assay example 1 | 50 |
| lignin sulfonate | 5 |
| alkyl sulfonate | 3 |
| diatomaceous earth | 42 |

The above components were ground and mixed to prepare water dispesible powder, which can be used by dilution with water.

Preparative example 3

Granules were prepared from the following composition.

| | Parts by weight |
|---|---|
| Compound (c) in Assay example 1 | 5 |
| bentonite | 43 |
| clay | 45 |
| lignin sulfonate | 7 |

The above components were mixed uniformly, and water was further added. The whole was kneaded and applied to an extrusion granulator to form a granular shape. Granules were obtained by drying.

Preparative example 4

Emulsifiable concentrate was prepared from the following composition.

| | Parts by weight |
|---|---|
| Compound (e) in Assay example 1 | 20 |
| polyoxyethylene alkyl aryl ether | 10 |
| polyoxyethylene sorbitan monolaurate | 3 |
| xylene | 67 |

The above components were mixed uniformly and dissolved to prepare emulsifiable concentrate.

INDUSTRIAL APPLICABILITY

According to the present invention, the yield of the esculetin synthesis is significantly improved in comparison with known methods. When one or more protecting groups are used, they can be removed easily. Therefore, the industrial value of the present invention is high.

Although the present invention has been described with reference to specific embodiments, various changes and modifications obvious to those skilled in the art are possible without departing from the scope of the appended claims.

**Claims**

**1.** A process for manufacturing an esculetin compound of the general formula (2):

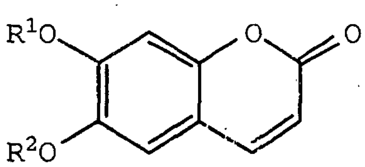

(2)

wherein $R^1$ and $R^2$ are, independently, a hydrogen atom, an alkyl group having 1 to 5 carbon atoms, an optionally substituted benzyl group, a formyl group, or an optionally substituted alkyl-carbonyl group in which the alkyl moiety has 1 to 4 carbon atoms; or $R^1$ and $R^2$ together form a $-CH_2-$ group or a $-C(CH_3)_2-$ group; **characterized by** cyclocondensing a trihydroxybenzaldehyde compound of the general formula (1):

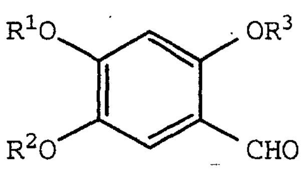

(1)

wherein $R^1$ and $R^2$ have the same meanings as above; and $R^3$ is a hydrogen atom, a formyl group, or an optionally substituted alkyl-carbonyl group in which the alkyl moiety has 1 to 4 carbon atoms, in an aprotic polar solvent in the presence of a weak base, with acetic anhydride or a compound which forms acetic anhydride in the reaction system.

**2.** A process for manufacturing a trihydroxybenzaldehyde compound of the general formula (1):

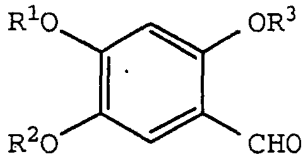

(1)

wherein $R^1$ and $R^2$ are, independently, a hydrogen atom, an alkyl group having 1 to 5 carbon atoms, an optionally substituted benzyl group, a formyl group, or an optionally substituted alkyl-carbonyl group in which the alkyl moiety has 1 to 4 carbon atoms, or $R^1$ and $R^2$ together form a $-CH_2-$ group or a $-C(CH_3)_2-$ group, and $R^3$ is a hydrogen atom, a formyl group, or an optionally substituted alkyl-carbonyl group in which the alkyl moiety has 1 to 4 carbon atoms, **characterized by** performing a formylation of a trihydroxybenzene compound of the general formula (3):

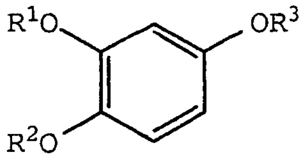

(3)

wherein $R^1$, $R^2$, and $R^3$ have the same meanings as above.

**3.** A trihydroxybenzaldehyde compound of the general formula (10):

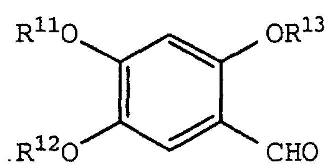

(10)

wherein $R^{11}$ and $R^{12}$ are, independently, a hydrogen atom or an alkyl group having 2 to 5 carbon atoms, or $R^{11}$ and $R^{12}$ together form a $-C(CH_3)_2-$ group, and $R^{13}$ is a hydrogen atom, a formyl group, or an optionally substituted alkyl-carbonyl group in which the alkyl moiety has 1 to 4 carbon atoms, with the proviso that $R^{11}$ and $R^{12}$ are not a hydrogen atom at the same time.

**4.** An esculetin compound of the general formula (20):

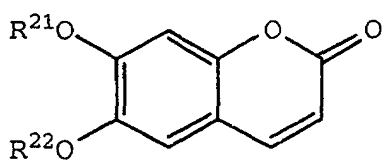

(20)

wherein $R^{21}$ and $R^{22}$ are, independently, a hydrogen atom, an alkyl group having 2 to 5 carbon atoms, an optionally substituted benzyl group, a formyl group, or an optionally substituted alkyl-carbonyl group in which the alkyl'moiety has 1 to 4 carbon atoms, or $R^{21}$ and $R^{22}$ together form a $-C(CH_3)_2-$ group, with the proviso that $R^{21}$ and $R^{22}$ are not a hydrogen atom at the same time, and that $R^{21}$ and $R^{22}$ are not a formyl group, or an optionally substituted

alkyl-carbonyl group in which the alkyl moiety has 1 to 4 carbon atoms at the same time.

**5.** A trihydroxybenzene compound of the general formula (30):

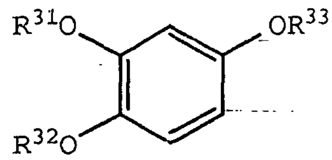

(30)

wherein $R^{31}$ is an optionally substituted benzyl group, $R^{32}$ is a hydrogen atom, a formyl group, or an optionally substituted alkyl-carbonyl group in which the alkyl moiety has 1 to 4 carbon atoms, or $R^{31}$ and $R^{32}$ together form a -$C(CH_3)_2$- group; and $R^{33}$ is a hydrogen atom, a formyl group, or an optionally substituted alkyl-carbonyl group, in which the alkyl moiety has 1 to 4 carbon atoms.

**6.** An antifungal composition for agriculture and horticulture, **characterized by** comprising, as an active ingredient, a trihydroxybenzaldehyde compound of the general formula (10):

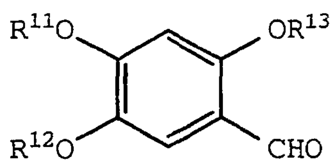

(10)

wherein $R^{11}$ and $R^{12}$ are, independently, a hydrogen atom or an alkyl group having 2 to 5 carbon atoms, or $R^{11}$ and $R^{12}$ together form a -$C(CH_3)_2$- group, and $R^{13}$ is a hydrogen atom, a formyl group, or an optionally substituted alkyl-carbonyl group in which the alkyl moiety has 1 to 4 carbon atoms, with the proviso that $R^{11}$ and $R^{12}$ are not a hydrogen atom at the same time.

**7.** An antifungal composition for agriculture and horticulture, **characterized by** comprising, as an active ingredient, an esculetin compound of the general formula (20):

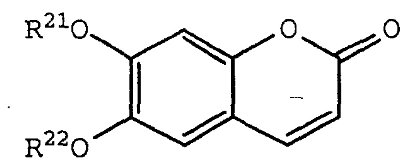

(20)

wherein $R^{21}$ and $R^{22}$ are, independently, a hydrogen atom, an alkyl group having 2 to 5 carbon atoms, an optionally substituted benzyl group, a formyl group, or an optionally substituted alkyl-carbonyl group in which the alkyl moiety has 1 to 4 carbon atoms, or $R^{21}$ and $R^{22}$ together form a -$C(CH_3)_2$- group, with the proviso that $R^{21}$ and $R^{22}$ are not a hydrogen atom at the same time, and that $R^{21}$ and $R^{22}$ are not a formyl group, or an optionally substituted alkyl-carbonyl group in which the alkyl moiety has 1 to 4 carbon atoms at the same time.

**8.** An antifungal composition for agriculture and horticulture, **characterized by** comprising, as an active ingredient, a trihydroxybenzene compound of the general formula (30):

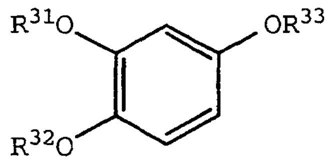

(30)

wherein $R^{31}$ is an optionally substituted benzyl group, $R^{32}$ is a hydrogen atom, a formyl group, or an optionally substituted alkyl-carbonyl group in which the alkyl moiety has 1 to 4 carbon atoms, or $R^{31}$ and $R^{32}$ together form a $-C(CH_3)_2-$ group, and $R^{33}$ is a hydrogen atom, a formyl group, or an optionally substituted alkyl-carbonyl group in which the alkyl moiety has 1 to 4 carbon atoms.

**9.** A herbicide **characterized by** comprising, as an active ingredient, a trihydroxybenzaldehyde compound of the general formula (10):

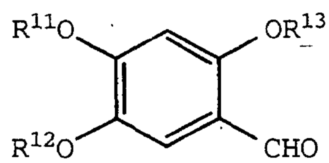

(10)

wherein $R^{11}$ and $R^{12}$ are, independently, a hydrogen atom or an alkyl group having 2 to 5 carbon atoms, or $R^{11}$ and $R^{12}$ together form a $-C(CH_3)_2-$ group, and $R^{13}$ is a hydrogen atom, a formyl group, or an optionally substituted alkyl-carbonyl group in which the alkyl moiety has 1 to 4 carbon atoms, with the proviso that $R^{11}$ and $R^{12}$ are not a hydrogen atom at the same time.

**10.** A herbicide **characterized by** comprising, as an active ingredient, an esculetin compound of the general formula (20):

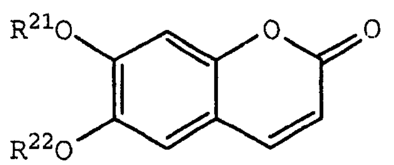

(20)

wherein $R^{21}$ and $R^{22}$ are, independently, a hydrogen atom, an alkyl group having 2 to 5 carbon atoms, an optionally substituted benzyl group, a formyl group, or an optionally substituted alkyl-carbonyl group in which the alkyl moiety has 1 to 4 carbon atoms, or $R^{21}$ and $R^{22}$ together form a $-C(CH_3)_2-$ group, with the proviso that $R^{21}$ and $R^{22}$ are not a hydrogen atom at the same time, and that $R^{21}$ and $R^{22}$ are not a formyl group, or an optionally substituted alkyl-carbonyl group in which the alkyl moiety has 1 to 4 carbon atoms at the same time.

**11.** A herbicide **characterized by** comprising, as an active ingredient, a trihydroxybenzene compound of the general formula (30):

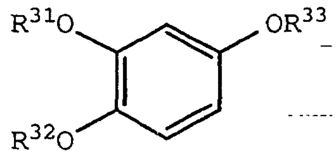

(30)

wherein $R^{31}$ is an optionally substituted benzyl group, $R^{32}$ is a hydrogen atom, a formyl group, or an optionally substituted alkyl-carbonyl group in which the alkyl moiety has 1 to 4 carbon atoms, or $R^{31}$ and $R^{32}$ together form a $-C(CH_{3)2}-$ group, and $R^{33}$ is a hydrogen atom, a formyl group, or an optionally substituted alkyl-carbonyl group in which the alkyl moiety has 1 to 4 carbon atoms.

## INTERNATIONAL SEARCH REPORT

International application No.
PCT/JP01/11633

**A. CLASSIFICATION OF SUBJECT MATTER**
Int.Cl[7] C07D311/16, C07C45/45, C07C47/575, C07C43/23, A01N43/16, A01N35/04, A01N37/02, A01N43/30
According to International Patent Classification (IPC) or to both national classification and IPC

**B. FIELDS SEARCHED**
Minimum documentation searched (classification system followed by classification symbols)
Int.Cl[7] C07D311/16, C07C45/45, C07C47/575, C07C43/23, A01N43/16, A01N35/04, A01N37/02, A01N43/30

Documentation searched other than minimum documentation to the extent that such documents are included in the fields searched

Electronic data base consulted during the international search (name of data base and, where practicable, search terms used)
CAPLUS(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP, 48-22456, A (Teikoku Chemical Industry Co., Ltd.), 22 March, 1973 (22.03.73), Particularly, Claims; Examples 2, 3 (Family: none) | 1-4<br>5,6,9 |
| X<br>A | ISHII, Hisashi et al., Synthetic Studies on Naturally Occurring Coumarins. II. Synthesis of 6, 7-Dimethoxy- and 7, 8-Dimethoxy-5-[(E)-3-oxo-1-butemyl] coumarins, Chem. Pharm. Bull., 1992, Vol.40, No.10, pages 2614 to 2619 | 2<br>1,2-6,<br>9 |
| X<br>A | MARI, R. S. et al., An Improved Synthesis of Naturally Occurring Coumarins: Synthesis of Herniarin, Scoparone & 7- Methoxy-6- methylcoumarin, Indian J. Chem., Sect. B, 1982, Vol.21B, pages 759 to 760 | 2<br>1,2-6,<br>9 |
| X<br>A | LU, A. Y. H. et al., ISOTOPE EFFECT AND METABOLIC SWITCHING IN THE CYTOCHROME P-450-CATALYZED O-DEETHYLATION OF 7-ETHOXYCOUMARIN, Dev. Biochem., 1982, Vol.23, pages 405 to 412 | 4<br>1-3,5,<br>6,9 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 April, 2002 (02.04.02) | 16 April, 2002 (16.04.02) |
| Name and mailing address of the ISA/<br>Japanese Patent Office<br>Facsimile No. | Authorized officer<br><br>Telephone No. |

Form PCT/ISA/210 (second sheet) (July 1998)

**INTERNATIONAL SEARCH REPORT**

International application No.
PCT/JP01/11633

C (Continuation). DOCUMENTS CONSIDERED TO BE RELEVANT

| Category* | Citation of document, with indication, where appropriate, of the relevant passages | Relevant to claim No. |
|---|---|---|
| X<br>A | THAKUR, R. S. et al., Hypotensive activity of some dihydroxycoumarins and their congeners, Experientia, 1978, Vol.34, No.2, pages 158 to 159 | 4<br>1- 3,5,6,9 |
| X<br>A | HARADA, Nobuhiro et al., Kinetic Isotope Effect on Cytochrome P-450-catalyzed Oxidation Reactions, J. Biol. Chem., 1984, Vol.259, No.5, pages 3005 to 3010 | 4<br>1- 3,5,6,9 |
| A | JP, 59-157003, A (Rikagaku Kenkyusho),<br>06 September, 1984 (06.09.84),<br>(Family: none) | 1- 6,9 |
| A | EP, 694257, A1 (American Cyanamid Co.),<br>31 January, 1996 (31.01.96),<br>& JP 8-73304 A & NO 9502940 A<br>& CZ 9501831 A & AU 9527151 A<br>& SK 9500929 A & CA 2154408 A<br>& BR 9503417 A & US 5563280 A<br>& ZA 9506151 A & CN 1119188 A<br>& NZ 272626 A & TW 318846 A | 1-6,9 |

Form PCT/ISA/210 (continuation of second sheet) (July 1998)

## INTERNATIONAL SEARCH REPORT

International application No.
PCT/JP01/11633

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
(See extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-6, 9

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP01/11633

Continuation of Box No.II of continuation of first sheet (1)

Claim 3 relates to compounds of the general formula (10). When claim 3 is regarded as the specified invention, claim 1 relates to a process for preparing compounds of the general formula (2) by using the compounds (10) as raw material, and claim 4 relates to products of the process. Further, claim 2 relates to a process for preparation of the above compounds, and claim 5 relates to starting compounds for the preparation. Additionally, claims 6 and 9 relate to antimould compositions and herbicides for agricultural and horticultural use, containing the compounds (10) as the active ingredient.

On the other hand, claims 7, 8, 10, and 11 relate to antimould compositions and herbicides for agricultural and horticultural use, containing as the active ingredient compounds of the general formula (20) or (30), and therefore have no relation to the compounds (10).

As described above, there is no technical relationship between claim 1 and a group of inventions of claims 7, 8, 10, and 11 involving one or more of the same or corresponding special technical features, and claim 1 and a group of inventions of claims 7, 8, 10, and 11 are not so linked as to form a single general inventive concept.

Form PCT/ISA/210 (extra sheet) (July 1998)